(19) Europäisches Patentamt
European Patent Office
Office européen des brevets

(11) Publication number: **0 272 483 A1**

(12) **EUROPEAN PATENT APPLICATION**

(21) Application number: **87117370.4**

(22) Date of filing: **25.11.87**

(51) Int. Cl.[4]: **C12N 15/00**, G01N 33/576, A61K 39/29

(30) Priority: **19.12.86 US 944645**

(43) Date of publication of application: **29.06.88 Bulletin 88/26**

(84) Designated Contracting States: **AT BE CH DE ES FR GB GR IT LI LU NL SE**

(71) Applicant: **ABBOTT LABORATORIES**
**Abbott Park Illinois 60064(US)**

(72) Inventor: **Andersen, Philip R.**
**235 Gillett Avenue**
**Waukegan Illinois 60085(US)**
Inventor: **Mushahwar, Isa K.**
**1923 Arthur Drive**
**Waukegan Illinois 60087(US)**
Inventor: **Mimms, Larry T.**
**8 Shoshoni Trail**
**Lake Villa Illinois 60046(US)**
Inventor: **Staller, Jonathan M.**
**105 Creekside Trial**
**McHenry Illinois 60050(US)**

(74) Representative: **Modiano, Guido et al**
**MODIANO, JOSIF, PISANTY & STAUB**
**Modiano & Associati Via Meravigli, 16**
**I-20123 Milan(IT)**

(54) **Methods and materials for HBeAg production.**

(57) Polynucleotides are disclosed which consist essentially of nucleic acids encoding polypeptides having HBeAg and HBcAg immunoreactivity, which are rendered substantially free of HBcAg immunoreactivity and which retain HBeAg immunoreactivity when denatured with a chaotrope and rapidly diluted into a non-denaturing buffer. Polypeptides which are expression products of bacterial, yeast or mammalian cells transformed with biologically functional DNA transformation vectors including such polynucleotides are also disclosed. These polypeptides may be used directly in immunoassays, may be bound to a reporter group or to a support and used in such assays, or may be used to raise polyclonal and monoclonal antibodies or as vaccine products.

EP 0 272 483 A1

# METHODS AND MATERIALS FOR HBeAg PRODUCTION

## BACKGROUND

The present invention relates in general to materials and methods for hepatitis B e antigen ("HBeAg") production by recombinant DNA methods. In particular, the present invention relates to HBeAg derived from DNA encoding polypeptides having HBeAg activity, and to methods for purification and derivation of proper epitopes by denaturation and dilution of the recombinant HBeAg.

The hepatitis B virus ("HBV") causes a disease now known as hepatitis B. formerly known as "serum hepatitis." It has been estimated that worldwide there are more than 200,000,000 people who are carriers of HBV. Infection with the virus is a major cause of acute and/or chronic liver disease. Carriers of hepatitis B virus have a high risk of contracting cirrhosis and hepatocellular carcinoma.

HBV has been identified in human serum as a "Dane" particle. The Dane particle is 42 nanometers in diameter and contains lipids, DNA, and at least four proteins, namely, hepatitis B surface antigen ("HBsAg"), hepatitis B core antigen ("HBcAg"), HBeAg and DNA polymerase.

HBeAg is part of the core of Dane particles, is a major constituent polypeptide and occurs either as described above or in association with immunoglobulin G ("IgG") in the serum of persons infected with HBV [Imai et al., J. Immunol., 128, 69-72 (1982)]. The presence of HBeAg in a person's serum is associated with high infectivity and may be of prognostic value in predicting the course of liver disease [MacKay et al., J. Med. Virol., 8, 237-243 (1983)].

Both HBcAg and HBeAg are associated with the core of the Dane particle. [Takahashi et al., J. Immunol., 22, 275-279 (1979)], and are encoded in one region of the HBV genome. [Roosinck et al., Mol. Cell. Biol., 6, 1393-1400 (1986)]. Thus, despite the usefulness of HBeAg as a prognostic indicator, it is difficult to produce an HBeAg preparation that is free of HBsAg and HBcAg activity for direct use as a reagent in immunoassays, or for raising polyclonal or monoclonal antibodies for use in such immunoassays.

The primary source of HBeAg is the serum of patients infected with HBV. Purification of HBeAg from serum is difficult because HBeAg is present in very low concentrations (<1 ng/ml) and it may aggregate with itself. with IgG, or with serum albumin so that it is molecularly heterogeneous with respect to molecular weight and charge [Yamade et al., J. Gen. Virol., 55 , 75-86 (1981)].

An affinity column is generally employed in the purification of serum HBeAg wherein anti-HBe serves as a ligand over which HBeAg-containing serum is circulated. HBeAg is then eluted using harsh conditions such as high salt or low pH. The eluted HBeAg is subjected to gel filtration [Imai et al.. J. Immunol., 128, 69-72 (1982)].

HBeAg may also be obtained by the conversion of HBcAg to HBeAg through the use of proteolytic enzymes, reducing agents, sonication, treatment with chaotropic agents, or gradient/centrifugation in CsCl [Ohori et al., Intervirology, 13, 74-82 (1980)]. The source of HBcAg for conversion is the postmortem livers of HBV-infected patients. To obtain HBeAg from liver-derived HBcAg, liver tissue must be extracted and pelleted on a CsCl gradient to obtain a pure HBcAg preparation. The HBcAg preparation is then treated with SDS and $\beta$-mercaptoethanol to yield HBeAg, [Ferns et al., J. Gen. Virol. 65, 899-908 (1984)]. However, the use of infected serum or tissue carries the risk that infectious material may be retained in antigenic preparations derived therefrom. Furthermore, contamination of HBeAg preparations with HBcAg and other HBV antigens may not be entirely avoided in such preparations.

Another way of obtaining HBeAg is through proteolytic digestion of recombinant HBcAg [European Patent Application No. 75395; and MacKay et al., supra]. However, HBeAg derived in this manner may be highly contaminated with HBcAg and may only be recovered in low quantity.

Expression of the core region of the HBV genome in mammalian cells with recombinant plasmid vectors may result in secretion of HBeAg. [Roosinck et al., supra; Bruss et al., Abstracts of Papers Presented at the 1986 Meeting on Molecular Biology of Hepatitis B Viruses (August 28-August 31, 1986), Cold Spring Harbor Laboratory, Cold Spring Harbor, New York, Abstract No. 14 (1982); and Ou et al., Abstracts of Papers Presented at the 1986 Meeting on Molecular Biology of Hepatitis B Viruses (August 28-August 31, 1986), Cold Spring Harbor Laboratory, Cold Spring Harbor, New York, Abstract No. 15 (1982)]. However, contamination with HBcAg is not eliminated by this approach.

It might be supposed that one approach to providing pure HBeAg may be to express HBeAg as a direct gene product of a recombinant HBcAg coding sequence from which non-HBeAg encoding regions have been trimmed (MacKay et al., supra) to leave only that sequence which encodes naturally-occurring HBeAg [Takahashi et al., J. Immunol., 130, 2903-2907 (1983)]. Although E. coli expression products of the HBeAg coding region may be produced [Ma et al., Abstracts of Papers Presented at the 1986 Meeting on Molecular Biology of Hepatitis B Viruses (August 28-August 31, 1986), Cold Spring Harbor Laboratory, Cold Spring Harbor, New York, Abstract No. 25], available products of this type may exhibit residual HBcAg activity which limits their usefulness.

Furthermore, although HBcAg activity may be lost upon treatment with SDS and 2-mercaptoethanol [Budkowska et al., J. Immunol. Methods, 51, 341-346 (1982)] and although other recombinant expression products may be properly renatured after purification and denaturation by rapid dilution [Prior et al., PCT Publication No. WO 85/05637], a process for selectively retaining HBeAg activity in the absence of HBcAg activity after denaturation and purification of recombinant HBeAg (rDNA HBeAg) is desirable.

## Summary of the Invention

Polynucleotides according to the present invention consist essentially of nucleic acids encoding polypeptides having HBeAg and HBcAg immunoreactivity, which are rendered substantially free of HBcAg immunoreactivity and which retain HBeAg immunoreactivity when denatured with a chaotrope and rapidly diluted into a non-denaturing buffer. A preferred polynucleotide of the invention is the polynucleotide as shown in Fig. 8. Polypeptides which are expression products of bacterial, yeast or mammalian cells transformed with biologically functional DNA transformation vectors including such polynucleotides are also preferred according to the present invention.

Polypeptides according to the present invention may be used directly in immunoassays, may be bound to a reporter group or to a support and used in such assays, or may be used to raise polyclonal and monoclonal antibodies or as vaccine products.

## Brief Description of the Drawings

Fig. 1 is a restriction endonuclease map of the hepatitis B viral DNA insert of plasmid pHBV-8;

Fig. 2 is a nucleotide sequence for the hepatitis B virus core gene of plasmid pHBV-8;

Fig. 3 is a flow chart illustrating the construction of recombinant DNA for HBcAg production;

Fig 4 is a flow chart illustrating the construction of clones expressing HBcAg;

Fig. 5 is a nucleotide sequence for the coding strand of clone 12.88b and a deduced amino acid sequence;

Fig. 6 is a flow chart illustrating the construction of deletion mutants of clone 12.88b;

Fig. 7 is a comparison of the deletion mutants construction with the original clone 12.88b;

Fig. 8 is a nucleotide sequence for clone 16.4 and a deduced amino acid sequence;

Fig. 9 is a frequency distribution plot for percent neutralization of and rDNA HBeAg according to the present invention in an anti-HBe RIA performed on an HBsAg-negative population;

Fig. 10 is a frequency distribution plot for percent neutralization of a human plasma-derived HBeAg in an anti-HBe RIA performed on an HBsAg-negative population;

Fig. 11 is a frequency distribution plot for percent neutralization of an rDNA HBeAg according to the present invention in an anti-HBe EIA performed on an HBsAg-negative population;

Fig. 12 is a frequency distribution plot for percent neutralization of a human plasma-derived HBeAg in an anti-HBe EIA performed on an HBsAg-negative population;

Fig. 13 is a frequency distribution plot for percent neutralization of a human plasma-derived HBeAg in an anti-HBe RIA performed on an HBsAg-positive population;

Fig. 14 is a frequency distribution plot for percent neutralization of an rDNA HBeAg according to the present invention in an anti-HBe RIA performed on an HBsAg-positive population;

Fig. 15 is a frequency distribution plot for percent neutralization of a human plasma-derived HBeAg in an anti-HBe EIA performed on an HBsAg-positive population; and

Fig. 16 is a frequency distribution plot for percent neutralization of an rDNA HBeAg according to the present invention in an anti-HBe EIA performed on an HBsAg-positive population.

## Detailed Description

The present invention is more specifically described in the following Examples. In Example 1, the construction of an HBcAg-producing clone is described. Example 2, illustrates the construction of deletion mutants of the HBcAg-producing clone of Example 1. Example 3, contains the results of assays for HBeAg expression products and the immunoreactivity of the deletion mutants of Example 2. Example 4 illustrates the production and isolation of HBeAg from a deletion mutant of Example 2. In Example 5, the purification and treatment for retention of HBeAg activity with substantially no HBcAg activity is described. In Example 6, the performance of rDNA HBeAg according to the present invention is compared with the performance of human plasma-derived HBeAg in EIA and RIA neutralization assays. Example 7 is a description of various immunoassay configurations in which rDNA HBeAg according to the present invention may be employed. Example 8 is a description of the production of antisera and polyclonal antibodies according to the present invention, which antibodies are specific for HBeAg. In Example 9, the production of monoclonal antibodies specific for HBeAg according to the present invention is described.

## Example 1

To construct an HBeAg-producing clone it was necessary to develop constructions which were capable of HBcAg biosynthesis.

The cloning of HBV DNA isolated from Dane particles (isolated from the sera of chronically infected individuals) was performed generally as described by Valenzuela et al., Nature, 280, 815-819 (1979).

The DNA was labeled with $^{32}$P-ATP and $^{32}$P-CTP by the endogenous DNA polymerase reaction [Hruska et al., J. Virol., 23, 368-376 (1977)] and purified according to Landers et al., J. Virol., 23 , 368-76 (1977). DNA purified from the isolated materials was digested with endonuclease EcoRI to give a single EcoRI fragment of about 3200 base pairs (bp). This 3200 bp fragment was ligated into the EcoRI site of plasmid pBR322, and E. coli cells of strain chi-1776 were transformed with the ligation product. Colonies resulting from the transformation were screened for sensitivity to tetracycline [Bolivar et al., Gene, 2, 95-113 (1977)] and by analysis of their plasmids [Barnes, Science, 195, 393-394 (1977)].

To confirm that the material was cloned HBV and in order to localize genes coding for HBcAg, a detailed restriction endonuclease map was constructed for an HBV clone designate pHBV-8, as illustrated in Fig. 1. This map was constructed using appropriate single and double enzyme digests to determine the position of restriction sites. By comparison with published restriction endonuclease maps and sequencing data [Valenzuela et al., in Animal Virus Genetics, Jaenish et al. eds. Academic Press, 57-70 (1980); and Galibert et al. Nature, 281, 646-650 (1979)], regions of the viral DNA which could potentially code for either HBsAg or HBcAg were identified.

To determine whether the recombinant DNA clone contained an intact gene for HBcAg synthesis, the DNA sequence of the region thought to code for HBcAg (on the basis of restriction site homology with published maps and sequences previously cited) was determined according to the methods described by Maxam et al., Methods Enzymol., 68, 499-560 (1980). The nucleotide sequence for the cloned HBcAg portion of pHBV-8 is illustrated in Fig. 2. By comparison of the resulting DNA sequence to published sequences, it was determined that the coding region for HBcAg was intact. Therefore, the cloned HBV DNA was utilized in engineering plasmids for HBcAg synthesis in bacteria.

The recombinant DNA techniques utilized for construction of clones in this and later examples are described in detail in Molecular Cloning, A Laboratory Manual, Maniatis et al., Cold Spring Laboratory, 75-186 (1982). The molecular construction designed for the synthesis of HBcAg is schematically illustrated in the construction in Fig. 3. The construction was initiated with the digestion of the HBV DNA insert of clone pHBV-8 with the restriction endonuclease HhaI (New England Biolabs, Beverly, Massachusetts). The digestion was carried out in the presence of 50mM Tris-HCl (pH 8.0), 5 mM $MgCl_2$ and 0.5 mM dithiothreitol, according to the manufacturer's directions. A 1 kbp DNA fragment resulting from this digest was shown to contain the entire nucleotide sequence for HBcAg by restriction endonuclease mapping and nucleic acid sequence analysis, and by comparison of the nucleotide sequence to published sequence data (Valenzuela et al. supra).

In order to obtain a substantial amount of a recombinant protein, cloned DNA may be fused to a plasmid-borne gene encoding $\beta$-galactosidase. This fusion is generated through the use of a "universal cloning site" within the region coding for an $\alpha$ subunit of $\beta$-galactosidase. Fusions between $\beta$-galactosidase and HBcAg are stable [Stahl et al., Proc. Nat'l. Acad. Sci. (USA), 79, 1606-1610 (1982)].

The plasmid pUC9 (P.L. Biochemicals, Milwaukee, Wisconsin) was selected as the expression vector.

This plasmid is capable of maintaining at least 10 to 15 copies of the gene to be expressed in each cell. The plasmid contains the genes required for β-galactosidase synthesis in E. coli bacterial strains JM103 and JM83 (both of which strains are available from P.L. Biochemicals, Milwaukee, Wisconsin).

The i gene (within the genome of the bacterial host JM103 or JM83) may be used to control expression of genes which are transcribed from the β-galactosidase promoter ($P_{lac}$). Where a protein product is toxic to the host, this form of control is extremely useful.

Flanking either side of the HBcAg coding region are noncoding hepatitis DNA sequences. Therefore, these noncoding regions were carefully trimmed back in order to obtain maximal HBcAg production. In order to do this an HhaI DNA fragment was submitted to nuclease BAL31 digestion (see Fig. 4). This enzyme removes bases from either end of the DNA molecule such that DNA fragments of various lengths are obtained. BAL31 (New England Biolabs) was used according to the manufacturer's directions and digestion was carried out in the presence of 600 mM NaCl, 12 mM $CaCl_2$, 12 mM $MgCl_2$, 20 mM Tris-HCl pH 8.0, and 1.0 mM EDTA. To monitor the extent of digestion, the reactions were stopped at 1, 2, 4, 6 and 8 minutes by addition of EDTA to 33 mM and phenol extraction. Products were analyzed by electrophoresis on agarose gels.

To ensure that the molecules generated by nuclease BAL31 treatment have blunt ends, they are treated with mung bean nuclease, a single strand specific endonuclease, and T4 DNA polymerase, an enzyme which can generate blunt-ended DNA molecules. Mung bean nuclease (P.L. Biochemicals, Milwaukee, Wisconsin) was utilized according to the manufacturer's directions and digestion of BAL31 treated DNAs were performed in the presence of 20 mM sodium acetate (pH 4.6), 50 mM NaCl, 1 mM zinc sulphate for 30 minutes at 37°C. The products of the reaction were then phenol extracted and ethanol precipitated. These products, after recovery by centrifugation and dissolving in water, were then treated with T4 DNA polymerase (P.L. Biochemicals) according to the manufacturer's directions. Reactions were carried out in the presence of 67 mM Tris-acetate pH 6.7, 10 mM $MgCl_2$, 5 mM dithiothreitol, 50 ug/ml BSA, and 33 mM each dTTP, dCTP, dGTP, dATP. Reaction mixtures were incubated for 30 minutes at 15°C and for 10 minutes at 37°C. Products in the reaction mixtures were resolved by agarose gel electrophoresis and the fragments between 700 and 900 base pairs in size were pooled by electroelution and harvested by ethanol precipitation.

The pooled harvested HhaI treated fragments were then combined with pUC9 DNA (Bethesda Research Labs, Gaitherburg, Maryland) cut with SmaI in an equimolar ratio. The DNAs were reprecipitated with ethanol, then pelleted and briefly lyophilized. The sample was dissolved in water and brought to a final concentration of 50 mM Tris-HCl pH 7.5, 20 mM dithiothrietol, 1 mM ATP, 10 Mm $MgCl_2$. T4 DNA ligase (P.L. Biochemicals, Milwaukee, Wisconsin) was then added and ligation was performed at 4°C for 48 hours.

The resultant ligated DNA molecules were used to tranfect the E. coli bacterial strain JM83 utilizing a modification of the technique described by Mandel et al., J. Mol. Biol., 53 , 154 (1970). Briefly, 0.5 ml of an overnight culture of JM83 cells were inoculated into 50 ml of fresh NZY media (N-Z Amine type A, Humko Sheffield, Memphis, Tennessee, (10 grams), NaCl (5 grams), $MgCl_2 7H_2O$ (2 grams), yeast extract (5 grams) in one liter of distilled water, [pH is adjusted to 7.0 with 6N NaOH] and allowed to grow for 2 hours at 37°C. Bacterial cells were then pelleted and washed twice in 25 ml of 10 mM NaCl. The final pellet was resuspended in 30 mM $CaCl_2$ (25 ml) and left on an ice bath for 30 minutes. The cells were pelleted and resuspended in 1.5 ml of 30 mM $CaCl_2$. The cells were then competent for transformation. To 200 ul of the cell suspension, 1 ug of ligated DNA was added. The cells were left on ice for 5 minutes, then heat shocked by incubation at 42°C for 2 minutes. To each transfection 2.5 ml of NZY media was added and cells were incubated for 1.5 hours at 37°C. The cells were spread on plates containing NZY media plus 1.5% agar and 40 ug/ml ampicillin. The plates were allowed to incubate overnight at 37°C. Bacterial colonies were then screened for insertion of the HBcAg gene by colony hybridization [Maniatis et al., supra (1982)]. A BglII fragment which identified only HBcAg coding sequences was labeled with $^{32}P$ by nick translation using a kit for this purpose (Amersham, Arlington Heights, Illinois). This probe was utilized to identify bacterial colonies for further study.

Individual bacterial colonies were grown overnight in 20 ml cultures of NZY media in the presence of IPTG [$4 \times 10^{4}$ M] (Bethesda Research Labs), a chemical which induces expression of genes under control of the lac promoter. Bacterial cell lysates were prepared [Stahl et al., Proc. Natl. Acad. Sci. (USA), 79, 1606-1610 (1982)] as follows. Cells were resuspended in 0.075 ml of 25% sucrose and 50 mM Tris buffer (pH 8). Twenty-five microliters of 0.25 M Tris buffer (pH 8) and 0.25 M EDTA were added, and the cells were placed in an ice water bath for 5 minutes. Next 0.12 ml of a solution of 1% Triton X-100® detergent (Sigma Chemical Company), 0.4% sodium deoxycholate, 50 mM Tris buffer (pH 8), and 62.5 mM EDTA was added before placing the resultant cell suspension in an ice water bath for 10 minutes at 0°C.

The cell suspension was sonicated at full power 3 times for 30 seconds. The sonicate was clarified by centrifugation at 30,000 rpm for 30 minutes in a 75 Ti rotor (Beckmann Industries, Palo Alto. California).

Clarified sonicates derived from individual bacterial colonies (clones) containing plasmids having an HBcAg gene insert were screened immunologically for HBcAg activity by the following method. 0.2 ml of a sample of clarified bacterial lysate was added to each of several assay wells. Anti-HBc-coated beads (Abbott Laboratories) were added to the wells and incubated for 24 hours at room temperature. These beads were then washed three times with water 0.2 ml of $^{125}$I-labeled anti-HBc (radioactivity maximum 7.7 uCi/ml; available from Abbott Laboratories) was added to each well and incubated for 4 hours at 45HBcAg C. Next the bead was washed 3 times with water and gamma emissions were counted in a gamma counter (ANSR® instrument, Abbott Laboratories).

A number of bacterial colonies were found to be capable of HBcAg biosynthesis in bacteria. When different clones were screened, as much as a 200-fold difference in the level of HBcAg production was found. This difference was found to be dependent on the primary structure of the construction, which differed in each clone. The clone producing the highest level of HBcAg was designated 12.88b. The primary structure of the coding strand of clone 12.88b was determined by DNA sequencing [Maxam et al., Methods Enzymol., 68, 499-560 (1980)] and is presented in Fig. 3. The amino acid sequence of the HBcAg protein fusion peptide, as deduced from the nucleic acid sequence, is shown in Fig.5.

## Example 2

Deletion mutants of the HBcAg-producing clone were constructed as shown in Fig. 6. Three series of constructs were developed and their relationship to clone 12.88b is illustrated in Fig. 7. Restriction digests were performed according to the manufacturer's instructions, and ligations were performed as described in Example 1.

Series 15 and 16 constructs were prepared by isolating plasmids (according to the procedure of Example 1) from clone 12.88b and by subjecting the plasmids to Hae III digestion.

Clone 15 series plasmids were then derived by restriction endonuclease cleavage of the HaeIII fragment with RsaI (New England Biolabs) which made a blunt ended cut within the HBcAg gene. The DNA fragment was then digested with the restriction endonuclease HindIII Bethesda Research Labs) and then ligated directly into SmaI and HindIII-cut pUC9 plasmid DNA.

Similarly, clone 16 series plasmids were obtained by digestion of the isolated HaeIII fragment with HpaII. Because HpaII digests give rise to staggered termini on DNA molecules, the termini of the series 16 HpaII fragments were filled in with T4 DNA polymerase to provide blunt ended molecules.

The blunt-ended molecules from both the series 15 and the series 16 digests were cut with HindIII and then ligated to HindIII and SmaI-cut pUC9 plasmid DNA, which was used to transform cells of E. coli strain JM83.

Clone 18 series plasmids represent an internal deletion of HBcAg DNA sequences from clone 12.88b. Clone 12.88b was digested with Bg III. The largest fragment from this digest contained the pUC9 plasmid DNA and HBcAg DNA having internal core coding sequences deleted. When this linear fragment was recircularized, ligated and used to transform cells of E. coli strain JM83 as described in Example 1, 100 bp of the coding sequence from the 5′ end of the HBcAg gene and 30 bp of the 3′ end of the HBcAg gene were transcribed into a single mRNA. Because the 3′ end of the coding sequence is in frame with the 5′ coding sequence based on the sequence for clone 12.88b as shown in Fig. 5, the protein coded for by that region has both the amino terminal and the carboxy terminal amino acids of HBcAg.

Following transformation of E. coli strain JM83 with clone 15, 16 and 18 series plasmids, transformants were screened by colony hybridization following the procedure of Example 1. Those colonies which hybridized to the HBcAg DNA specific-probe described in Example 1 were then analyzed for HBcAg immunorectivity.

## Example 3

Individual bacterial colonies containing cells having plasmids of one of each of the series were grown in NZY medium overnight in the presence of 4 $\times$ $10^4$ M isopropylthio-$\beta$-galactoside (IPTG), a chemical which induces expression of a gene under control of the lac promoter. Bacterial cell lysates were prepared as described in Example 1, and then analyzed for both HBcAg and HBeAg as generally described in Example 5.

The HBcAg assay was performed as described in Example 1. The results of this assay are presented in Table 1.

In the HBeAg assay, 0.2 ml of a sample of clarified bacterial lysate was added to each of several assay wells. Anti-HBe coated beads (Abbott Laboratories) were added to the wells and incubated for 24 hours at room temperature. The beads were then washed 3 times with water before adding 0.2 ml of $^{125}$I-labeled anti-HBe (radioactivity maximum 3.8 uCi/ml, Abbott Laboratories) to each well. The results of this assay are also presented in Table 1.

Table 1

| | HBcAg Test c.p.m. | HBeAg Test c.p.m. |
|---|---|---|
| Clone 15.3 | 28,000 | 46,000 |
| Clone 15.8 | 24,000 | 15,000 |
| Clone 15.10 | 6,000 | 9,000 |
| Clone 16.2 | 118,442 | 172,830 |
| Clone 16.4 | 89,206 | 163,280 |
| Clone 18 | 3,500 | 7,200 |
| Clone 12.88b | 120,424 | 30,000 |
| Neg. Control (PBS + 10% fetal calf serum) | 500 | 300 |

All clones of each series reacted in both assays. As would be expected, there was less reactivity in the HBcAg assay as less and less HBcAg coding sequence remained in the expression plasmid. The relative reactivities of the expression products of clones 16.2 and 16.4 in the HBcAg and HBeAg assay indicate that these two clones may express a polypeptide similar to naturally-occurring HBeAg.

Clone 16.4 was sequenced and the results are presented in Fig. 8. When the DNA sequence for clone 16.4 was analyzed it was found that 120 base pairs of HBcAg coding sequence had been deleted from the 3′ end. This resulted in a loss of 40 HBcAg coded amino acids from the carboxy-terminus of the protein and an addition of 9 amino acids as a result of the fusion with bacterial plasmid sequences. Following construction of clone 16.4, the carboxy-terminal sequence of naturally-occurring HBeAg was determined [Takahashi et al, J. Immunol., 130, 2903-2907 (1983)]. The carboxy-terminal amino acids of HBeAg are -Thr-Thr-Val-Val, or -Thr-Thr, while the carboxy-terminal amino acids of HBeAg coded for in clone 16.4 are -Leu-Pro-Glu. These amino acids are immediately adjacent to the -Thr-Thr found in naturally-occurring HBeAg. Therefore, the antigen produced by clone 16.4 lacks 2 to 4 amino acids found in the naturally occurring HBeAg.

Conversion of HBcAg to HBeAg in nature appears to be a two-step procedure, involving proteolytic cleavage of the antigen and alteration of the protein's tertiary structure. The proteolytic cleavage step has been replaced in the present invention by deleting HBcAg coding sequences. The deletion mutant clone 16.4 produces a protein much like that of naturally occurring HBeAg except that the protein is fused to the $\alpha$ subunit of $\beta$-galactosidase and exists as an aggregated complex when extracted from antigen-producing cells. Denaturation of these molecular aggregates appears to be required in order for the molecules to take on a true HBeAg character. This effect of denaturation is apparent from a comparison of treatment of clone 12.88b antigen and clone 16.2 antigen to various denaturing conditions.

Equivalent amounts of bacterial antigen (prepared as previously described) from clone 12.88b and clone 16.2, were aliquoted into test tubes and adjusted to the final salt and buffer concentrations desired. Tubes submitted to treatment I contained 10 mM Tris-HCl pH 7.5, 100 mM NaCl, and 1 mM EDTA. Tubes submitted to treatment II contained 50 mM Tris-HCl, pH 8.5, 8M GuHCl, 6 mM EDTA, 6mM dithiothrietol. Tubes submitted to treatment III contained 10 mM Tris-HCl pH 7.5, 100 mM NaCl, 1 mM EDTA 0.1% sodium dodecyl sulfate and 10 mM $\beta$-mercaptoethanol. The antigens in these buffers were then heated at 45°C for 30 minutes. The preparations were then dialyzed exhaustively against a buffer containing 10 mM Tris-HCl pH 7.5 100 mM NaCl, and 1 mM EDTA. Antigen preparations were then analyzed for their reactivity in HBcAg and HBeAg assays as previously described; all results are normalized with respect to treatment I, which is a buffer exposing the antigen to nondenaturing condition. The results are presented in Table 2.

## Table 2

### Effect of Denaturation On The Reactivity of rDNA HBcAg And HBeAg In HBcAg And HBeAg Immunoassays

| | Clone 12.88b Antigen | | Clone 16.2 Antigen | |
|---|---|---|---|---|
| Treatment | HBcAg Assay | HBeAg Assay | HBcAg Assay | HBeAg Assay |
| I | 100% | 100% | 100% | 100% |
| II | 113% | 130% | 8% | 103% |
| III | 124% | 146% | 18% | 107% |

The results indicate that the deletion of HBcAg gene sequences is insufficient to generate a purely HBeAg reactive molecule, since subjection of clone 12.88b and 16.2 specified proteins to nondenaturing conditions resulted in proteins reactive in both HBcAg and HBeAg assays. Furthermore, subjecting the entire HBcAg gene product of clone 12.88b to denaturing conditions fails to eliminate the HBcAg reactive epitopes on the molecule, as shown by treatments II and III on clone 12.88b and the reactivities in HBcAg and HBeAg assays. Only treatment of the deletion mutant 16.2 with denaturants provided material that was preferentially reactive in the HBeAg assay. These results were the first indication that solely HBeAg-reactive molecules could be generated by deletion and denaturation.

### Example 4

Growth of clone 16.4 and isolation of HBeAg was accomplished as follows. Five 4-liter flasks, each containing 2 liters of NZY + Thiamine broth (Difco, Detroit, Michigan), were prepared and autoclaved according to the manufacturer's instruction. Upon cooling of the media, 200 mg of ampicillin and 190 mg of IPTG (Sigma Chemical Company, St. Louis, Missouri) were aseptically added to each flask and swirled to mix.

From an overnight started culture, 15 ml of E. coli was added to each flask, and swirled at 200 ± 25 rpm and at 37°C for 22 ± 6 hours. After incubation, the 5 flasks were visually noted to contain significant bacterial biomass. The bacteria were pelleted at 10,410 X g for 20 minutes at 2-8°C, and the spent media was discarded. The pellet was placed on ice for the remainder of the procedure. The pellets were resuspended in a total volume of 300 ml M9 minimal salts solution (Difco, Detroit, Michigan) plus 2% glucose and cooled. Again the bacterial suspension was pelleted as above and the supernatant was discarded.

A solution containing 67.5 ml of 0.05 M Tris (pH 8.0) plus 25% sucrose buffer was added to the bacterial pellet and the pellet was resuspended with a Pasteur pipette to break up clumps. A buffer consisting of 0.25 M Tris and 0.25 M EDTA (pH 8.0) was prepared, and 25 ml was added to the bacterial suspension. The suspension was swirled and left on ice for a 10 minute incubation. A lysis buffer [0.5 mM Tris, 1% Triton X-100®, 0.01 M EDTA, 1 mg Aprotinin, and 22 mg phenylmethyl sulfonyl fluoride (PMSF)] was precooled and 108 ml of the lysis buffer was added to the above bacterial suspension. The solution was sonicated 10 times for 30 seconds each with a Vibra-Cel sonifier (Science and Materials, Inc., Danbury, Connecticut) waiting 1 or 2 minutes between each cycle.

The sonicated bacterial suspension was distributed to centrifuge tubes and spun at 135,000 X g for 30 minutes to clarify the solution. At the end of the run, the clarified lysate containing the recombinant protein was decanted and saved while the pellet was discarded. The lysate was placed on a linear sucrose gradient (0% to 60%) and was centrifuged for 18 to 24 hours. Fractions were collected and assayed for the presence of HBeAg activity with a commercially available diagnostic test kit (available as Abbott-HBe from Abbott Laboratories). The fractions with the highest HBeAg activities were pooled and stored until the denaturation step. The resulting sucrose gradient pool exhibits significant HBeAg activity as well as a moderate level of HBcAg activity as tested by the previously described HBcAg assay.

The following denaturation and dilution procedure produces a polypeptide product having high HBeAg activity and lacking HBcAg activity. This process involves denaturation of the recombinant protein in a chaotropic agent, preferably, 8 M guanidine hydrochloride (GuHCl) followed by rapid dilution in a non-denaturing agent, preferably, human plasma.

Example 5

52 ml of 10 M GuHCl in 50 mM Tris (pH 8.5) was added to 4.0 ml of 0.1 M EDTA in distilled water, 4.0 ml of 0.1 M dithiothrietol (DTT) in distilled water, and 5.0 ml of the sucrose pool of clone 16.4. This solution was mixed and placed in a 45°C waterbath and incubated at this temperature for 30 minutes. The denatured HBeAg solution is immediately diluted 1:100 into human plasma.

As illustrated by the results presented in Table 3, dialysis of the denatured protein solution results in an increase of HBcAg activity. Therefore, dialysis is not an appropriate method for obtaining material having significant HBeAg reactivity which is substantially free of HBcAg reactivity. A direct 1:100 dilution of the material should be used.

Table 3

SPECIFIC ACTIVITIES
(Antigen Activity/mg Protein)

| | HBeAg activity | HBcAg activity |
|---|---|---|
| Before Denaturation | $4.2 \times 10^4$ | $1.2 \times 10^4$ |
| Undialyzed Material Treated by Quick Dilution 1:100 After Denaturation | $3.84 \times 10^9$ | -0- |
| Dialyzed Material After Denaturation | $7.72 \times 10^9$ | $1.06 \times 10^9$ |

In Table 3, antigen activity is measured as the resulting $A_{492}$ or cpm multiplied by the dilution factor of the sample. The assay performed before denaturation was an enzyme immunoassay (EIA) and the results are shown as $A_{492}$ multiplied by the dilution factor. The assays performed after denaturation were radioimmunoassays and, therefore, the results are stated as cpm multiplied by the dilution factor of the sample. The "-O-" value given for the HBcAg activity assay of the undialysed material indicates that the cpm of the sample was equivalent to the background signal of the assay.

In general, the effectiveness of particular conditions for renaturation of an antigen according to the present invention may be determined by the presence of HBeAg activity in an HBeAg assay of the sort described in Mushahwar et al., J. Med. Virol., 2, 77 (1978) accompanied by substantially no HBcAg activity for the same material when tested in a HBcAg assay of the sort described in Purcell et al., Intervirology, 2, 231 (1973).

The HBcAg assay was performed as described: Purified IgG from human sera having a high anti-HBc titer but no detectable anti-HBe (determined by commercially available kits, Abbott Laboratories) was coated onto one-quarter inch polystyrene beads. The anti-HBcAg coating concentration was 10-30 mg/ml. These coated beads were incubated with specimen for 2 hours at 40°C or overnight at room temperature. The beads were washed then incubated with purified human anti-HBc radiolabeled with $^{125}I$ using chloramine T or conjugated to horseradish peroxidase by the method of Nakane, J. Histochem. Cytochem., 22, 1084 (1974). After incubation for 2 hours at 40°C, the bead is washed and counted for radioactivity in the RIA version or added to a solution containing OPD (o-phenylene diamine) and incubated for 30 minutes at room temperature in the EIA version. The OPD solution absorbance is determined in a spectrophotometer.

The rDNA HBeAg described in this example may be further diluted into negative human plasma and used as neutralizing reagent in anti-HBe immunoassays described below. The rDNA HBeAg may also be concentrated by standard protein concentration methods (i.e. vacuum dialysis).

Example 6

In both RIA and EIA versions of the anti-HBe test, beads coated with anti-HBe are simultaneously incubated with patient specimen and neutralizing reagent which contains HBeAg. The presence of anti-HBe in patient specimens will prevent the HBeAg in the neutralizing reagent from binding to the bead. After this first incubation, the unbound material is removed by washing the beads. The beads are then incubated in a second step with either $I^{125}$-labeled anti-HBe (RIA version) or horseradish peroxidase (HRPO) conjugated anti-HBe (EIA version). In the RIA version, the beads are washed and immediately counted for radioactivity. In the EIA version, the beads are incubated with an enzyme substrate to produce a color change. The absorbance values are then determined. Specimens with counts per minute or absorbance values equal to or less than a calculated cutoff value are considered reactive ("positive").

Currently, the source for an HBeAg neutralizing reagent is human plasma. The recombinant DNA-generated HBeAg ("rDNA HBeAg") produced in Example 5, is identical in immunogenicity to the HBeAg isolated from human plasma. Neutralizing reagent containing rDNA HBeAg can be substituted for human plasma-derived neutralizing reagent ("hpHBeAg") in an anti-HBe assay. Data show that use of rDNA HBeAg neutralizing reagent yields greater sensitivity than plasma-derived neutralizing reagent in both RIA and EIA versions of the anti-HBe test.

With specimens testing positive for Hepatitis B surface antigen (HBsAg) there was 98% agreement in anti-HBe RIA test and 99% agreement in anti-HBe EIA test when rDNA HBeAg neutralizing reagent was substituted for plasma-derived neutralizing reagent. All discrepant samples were negative for anti-HBe using the plasma-derived neutralizing reagent, but positive for anti-HBeAg using the rDNA HBeAg neutralizing reagent. These discrepancies result from the increased sensitivity of the test when rDNA HBeAg neutralizing reagent is used. Specificity was determined by testing HBeAg-negative specimens. Data shown 99.7% agreement in the anti-HBe EIA test between plasma-derived and rDNA HBeAg neutralizing reagent.

To demonstrate the specificity of rDNA HBeAg neutralizing reagent and its equivalence a plasma-derived neutralizing reagent, the frequency distribution of anti-HBe in approximately 200 samples of HBsAg-negative sera was examined.

The frequency distribution of anti-HBe reactive specimens in HBsAg negative sera are shown for an RIA (Abbott HBe, Abbott Laboratories) in figures 9 and 10, and for an EIA (Abbott HBe EIA, Abbott Laboratories) in figures 11 and 12. Data is plotted as frequency (number of specimens) versus percent neutralization. The percent neutralization for both tests is determined as follows wherein percent neutralization is abbreviated "%N," negative control mean is abbreviated "NCM," sample mean is abbreviated "SM," and positive control mean is abbreviated "PCM":

$$\% N = \frac{NCM - SM}{NCM - PCM} \times 100$$

Specimens with a percent neutralization of greater than 50% are considered reactive.

As shown in Table 4, in a population of 200 HBsAg negative sera tested by RIA, one sample was found to be reactive for anti-HBe when the rDNA HBeAg neutralizing reagent was employed. This sample was just above the cutoff. No sample was found to be reactive for anti-HBeAg with the plasma-derived neutralizing reagent. Overall agreement was 99.50%.

As also shown in Table 4, when 200 HBsAg negative specimens were tested with Abbott-HBe EIA, all samples were found to be negative using either neutralizing reagent, so that agreement was 100%.

Table 4

HBsAg Negative Population

| | | rDNA HBeAg positive | rDNA HBeAg negative |
|---|---|---|---|
| hpHBeAg | positive | 0 | 0 |
| | negative | 1 | 199 |

Agreement = 199/200 99.5%

| | | rDNA HBeAg positive | rDNA HBeAg negative |
|---|---|---|---|
| hpHBeAg | positive | 0 | 0 |
| | negative | 0 | 200 |

Agreement = 200/200 = 100%

These data demonstrate that the use of a rDNA HBeAg neutralizing reagent exhibit similar specificity is the hpHBeAg neutralizing reagent when evaluating negative populations.

The frequency distribution of anti-HBe in an HBsAg-positive population of 200 serum samples was determined in order to compare rDNA HBeAg neutralizing reagent and hpHBeAg neutralizing reagent. The frequency distribution of anti-HBe reactive specimens for an RIA and for an EIA using rDNA HBeAg neutralizing reagent and hpHBeAg neutralizing reagent are illustrated in figures 13, 14, 15 and 16 respectively.

In a population of HBsAg positive specimens tested by RIA, 64 specimens were found to be negative with the current neutralizing reagent. Using the rDNA HBeAg neutralizing reagent, 60 specimens were found to be negative. As shown in Table 5, this results in 98.0% Agreement between assays.

When testing the same population by EIA, 60 samples were found to be negative using the current neutralizing reagent. Using the rDNA HBeAg neutralizing reagent 58 were found to be negative. As also shown in Table 5, this results in 99.0% agreement between assays.

Table 5

HBsAg Positive Populations

| | | rDNA HBeAg positive | rDNA HBeAg negative |
|---|---|---|---|
| hpHBeAg | positive | 136 | 0 |
| | negative | 4 | 60 |

196/200 = 98.0% Agreement

| | | rDNA HBeAg positive | rDNA HBeAg negative |
|---|---|---|---|
| hpHBeAg | positive | 140 | 0 |
| | negative | 2 | 58 |

198/200 = 99.0% Agreement

These results indicate an overall agreement between rDNA HBeAg neutralizing reagent and hpHBeAg neutralizing reagent of 96.5%. All specimens testing positive using the hpHBeAg neutralizing reagent are also positive using the rDNA HBeAg neutralizing reagent. All discrepant samples test negative using the hpHBeAg neutralizing reagent, but test positive using the rDNA HBeAg neutralizing reagent. Because these specimens are known to be HBsAg positive, discrepancies between rDNA and hpHBeAg neutralizing reagent result from the increased sensitivity afforded through the use of the rDNA-derived neutralizing reagent.

Two-fold serial dilutions of five anti-HBe positive specimens were assayed by RIA and EIA using both hpHBeAg neutralizing reagent and the rDNA HBeAg neutralizing reagent.

The anti-HBe test using rDNA HBeAg neutralizing reagent shows equivalent or greater sensitivity than the anti-HBe test using the current neutralizing reagent for all six specimens as indicated in Table 6.

Table 6

Reciprocal Endpoint Dilutions Comparing rDNA HBeAg Neutralizing Reagent With Current hpHBeAg Neutralizing Reagent in Abbott anti-HBe RIA and EIA

| | Abbott anti-HBe RIA | | Abbott anti-HBe EIA | |
|---|---|---|---|---|
| Specimen | hpHBeAg | rDNA HBeAg | hpHBeAg | rDNA HBeAg |
| 1 | 1 | 4 | 2 | 8 |
| 2 | 2 | 2 | 1 | 4 |
| 3 | 8 | >16 | 4 | 8 |
| 4 | 1 | 2 | – | 2 |
| 5 | 4 | 4 | 1 | 8 |

These data show that the use of rDNA HBeAg in the neutralizing reagent allows greater sensitivity than the hpHBeAg neutralizing reagent.

The purification of recombinant DNA-derived HBeAg by the method described herein produces larger yields of highly purified HBeAg preparation in one purification step than may be obtained either by the purification of serum HBeAg or by production of HBeAg from HBcAg preparations through more complicated procedures.

A purified HBeAg preparation may be used to immunize animals for the production of polyclonal antisera or for the production of monoclonal antibodies. Further purified HBeAg preparation may be used as a vaccine for protection against hepatitis B viral infections. These antibodies may in turn be incorporated into conventional radioimmunoassays or enzyme immunoassays for the purpose of detecting the presence of HBeAg or its corresponding antibody (anti-HBe) in patients. A major advantage will be in the continued availability and uniformity of HBeAg preparations. Recombinant HBeAg produced and purified according to the present invention is very stable compared to the naturally-occurring HBeAg.

Example 7

Other Immunoassay Configurations Utilizing Recombinant HBeAg According to the Present Invention to Detect Antibody to HBeAg

Several immunoassays to detect anti-HBe may be developed using rDNA HBeAg. Three examples are given:

A) rDNA HBeAg may be used in a sandwich assay to detect antibody against HBeAg. In this assay, a solid phase (microtiter, polystyrene bead, microbeads) coated with rDNA HBeAg is incubated with specimen, washed and then reacted with a solution containing rDNA HBeAg which has been radiolabeled for RIA or conjugated to an enzyme for EIA.

B) rDNA HBeAg may be used in a competitive immunoassay in which rDNA HBeAg is coated on a solid phase and incubated with test sample and labeled anti-HBeAg antibody (either polyclonal or monoclonal). If antibody to HBeAg is present in test sample, it will compete against the binding of the labeled anti-HBe to the solid phase.

C) Recombinant HBeAg is coated on a solid phase which is incubated with a test sample (human serum). The solid phase is washed, and then incubated with labeled anti-human antibody, such as goat anti-human IgG or goat anti-human IgM.

Example 8

Purified rDNA HBeAg may be used to produce antisera.

Antisera may be specifically produced by immunizing rabbits with injections of purified polypeptides according to the present invention as follows. The first inoculation contains the antigen with Freund's complete adjuvant. Succeeding inoculations contained the antigens and 0.25 ml of Freund's incomplete adjuvant. The animals are bled to obtain sera. Polyclonal antibodies may be isolated from the sera by affinity chromatography.

Example 9

Monoclonal antibodies according to the present invention may be produced according to the procedure of Greenberg et al., Infect. Immun., 39, 91-99 (1983) with the substitution of a solution of concentrated rDNA HBeAg according to the present invention for the immunogen concentrate employed therein.

Basically, monoclonal antibodies are produced by injecting mice with immunizing doses of rDNA HBeAg protein, as described in Example 5. Spleens are removed from the immunized animals, and spleen cells are fused to myeloma cells (e.g. NS-1 cells) using a fusogen, such as polyethylene glycol. Hybridoma cells producing monoclonals are selected for in HAT medium. Monoclonal antibodies specific for rDNA HBeAg protein may be isolated by affinity chromatography from media in which such hybridomas have been cultured. These monoclonal and polyclonal antibodies may be used to develop HBeAg and anti-HBe immunoassays.

Although the present invention has been described in terms of a preferred embodiment, it is understood that variations and improvements will occur to those skilled in the art upon consideration of this disclosure. Accordingly, it is intended that the present invention include all such variations and modifications which come within the scope of the invention as claimed.

**Claims**

1. A polynucleotide comprising a nucleic acid sequence encoding a polypeptide having HBcAg and HBeAg immunoreactivity, which is rendered substantially free of HBcAg immunoreactivity but retains HBeAg immunoreactivity when denatured with a chaotrope and rapidly diluted into a nondenaturing buffer.

2. The polynucleotide of claim 1 wherein said polynucleotide comprises a nucleic acid sequence lacking 120 nucleotides from the 3′ end of the HBcAg coding sequence.

3. The polynucleotide of claim 1 wherein said polynucleotide has a nucleotide sequence as shown in Fig. 8.

4. The polypeptide expression product of the polynucleotide of claim 1, wherein said polypeptide is an E. Coli expression product.

5. The polynucleotide of claim 1 wherein said polynucleotide is a Bgl II digestion product of a polynucleotide having a nucleotide sequence shown in Fig. 5.

6. The polynucleotide of claim 1 wherein said polynucleotide is a HaeIII, RsaI and Hind III digestion product of a polynucleotide having a nucleotide sequence as shown in Fig. 5.

7. A polypeptide expression product of the polynucleotide of claims 1, 2, 3, 5 or 6.

8. A method of making a polypeptide having HBeAg immunoreactivity but lacking HBcAg immunoreactivity comprising:

a. expressing the polynucleotide of claim 1 to obtain a polypeptide having HBcAg and HBeAg immunoreactivity;

b. denaturing said polypeptide in a chaotropic agent; and

c. rapidly diluting said denatured protein in a non-denaturing agent.

9. A polypeptide made by the method of claim 8.

10. A method of making a polypeptide having HBeAg immunoreactivity but lacking HBcAg immunoreactivity comprising:

a. expressing the polynucleotide of claim 3 to obtain a polypeptide having HBcAg and HBeAg immunoreactivity;

b. denaturing said polypeptide in a chaotropic agent; and

c. rapidly diluting said denatured protein in a non-denaturing agent.

11. A polypeptide made by the method of claim 10.

12. The method of claim 8 or 10 wherein said chaotropic agent is guanadine hydrochloride.

13. The method of claim 8 or 10 wherein said non-denaturing agent is human plasma.

14. A biologically functional DNA microorganism transformation vector comprising a polynucleotide according to claim 1.

15. An assay for detection of antibody to HBeAg in a sample comprising contacting a solid support coated with anti-HBe with the sample and a neutralizing reagent comprising the polypeptide of claim 9, and then contacting the solid support with anti-HBe associated with a reporter group and detecting the presence of the reporter group on the solid support.

16. An assay for detection of antibody to HBeAg in a sample comprising exposing the sample to a solid support coated with the polypeptide of claim 9, and then contacting the solid support with the polypeptide of claim 10 associated with a reporter group, and detecting the presence of the reporter group on the solid support.

17. An assay for detection of antibody to HBeAg in a sample comprising contacting a solid support coated with the polypeptide of claim 9 with the sample and anti-HBe associated with a reporter group and detecting the presence of the reporter group on the solid support.

18. An assay for detection of antibody to HBeAg in a sample comprising contacting a solid support coated with the polypeptide of claim 9 with the sample, and then contacting the solid support with anti-human antibody associated with a reporter group and detecting the presence of the reporter group on the solid support.

19. A method of producing a polyclonal antibody against the polypeptide of claim 9 comprising immunizing an animal with an effective amount of the polypeptide of claim 9 and isolating anti-HBe antibodies from serum, plasma or other body fluid of the animal.

20. A polyclonal antibody produced according to the method of claim 19.

21. A method of producing a monoclonal antibody against the polypeptide of claim 13 comprising immunizing an animal with an effective amount of the polypeptide of claim 9, fusing antibody-producing cells from the animal with myeloma cells, isolating a hybridoma in a culture medium producing monoclonal antibodies against HBeAg and purifying the monoclonal antibody from the culture medium.

22. A monoclonal antibody produced according to the method of claim 21.

23. A vaccine comprising a pharmaceutically acceptable diluent, adjuvant or carrier and the polypeptide of claim 9.

FIG. 1

RESTRICTION ENDONUCLEASE MAP OF HBV DNA INSERT OF PLASMID pHBV-8

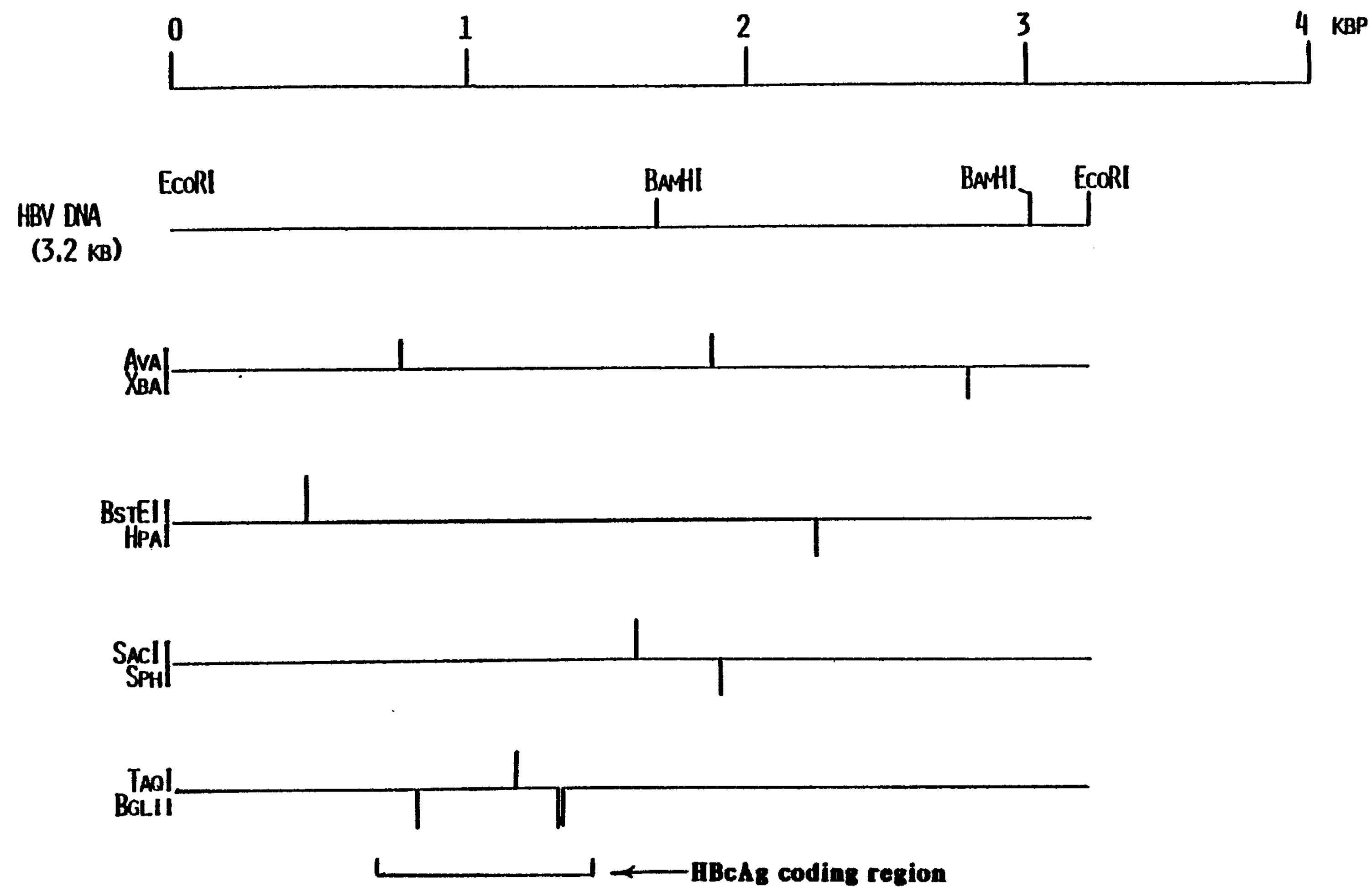

# FIG. 2

DNA AND AMINO ACID SEQUENCE OF HBV CORE GENEOF PLASMID pHBV-8

```
          -20                           1                             20
                                        MET GLN LEU PHE HIS LEU CYS LEU ILE ILE
TAG GCA TAA ATT GGT CTG CGC ACC AGC ACC ATG CAA CTT TTT CAC CTC TGC CTA ATC ATC

          40                            60                            80
SER CYS THR CYS PRO THR VAL GLN ALA SER LYS LEU CYS LEU GLY TRP LEU TRP GLY MET
TCT TGT ACA TGT CCC ACT GTT CAA GCC TCC AAG CTG TGC CTT GGG TGG CTT TGG GGC ATG

          100                           120                           140
ASP ILE ASP PRO TYR LYS GLU PHE GLY ALA THR VAL GLU LEU LEU SER PHE LEU PRO SER
GAC ATT GAC CCT TAT AAA GAA TTT GGA GCT ACT GTG GAG TTA CTC TCG TTT TTG CCT TCT

          160                           180                           200
ASP PHE PHE PRO SER VAL ARG ASP LEU LEU ASP THR ALA SER ALA LEU TYR ARG GLU ALA
GAC TTC TTT CCT TCC GTC AGA GAT CTC CTA GAC ACC GCC TCA GCT CTG TAT CGA GAA GCC

          220                           240                           260
LEU GLU SER PRO GLU HIS CYS SER PRO HIS HIS THR ALA LEU ARG GLN ALA ILE LEU CYS
TTA GAG TCT CCT GAG CAT TGC TCA CCT CAC CAT ACT GCA CTC AGG CAA GCC ATT CTC TGC

          280                           300                           320
TRP GLY GLU LEU MET THR LEU ALA THR TRP VAL GLY ASN ASN LEU GLU ASP PRO ALA SER
TGG GGG GAA TTG ATG ACT CTA GCT ACC TGG GTG GGC AAT AAT TTG GAA GAT CCA GCA TCT

          340                           360                           380
ARG ASP LEU VAL VAL ASN TYR VAL ASN THR ASN VAL GLY LEU LYS ILE ARG GLN LEU LEU
AGG GAC CTT GTA GTA AAT TAT GTT AAT ACT AAC GTG GGT TTA AAC ATC AGG CAA CTA TTG

          400                           420                           440
TRP PHE HIS ILE SER CYS LEU THR PHE GLY ARG GLU THR VAL LEU GLU TYR LEU VAL SER
TGG TTT CAT ATA TCT TGC CTT ACT TTT GGA AGA GAG ACT GTA CTT GAA TAT TTG GTC TCT

          460                           480                           500
PHE GLY VAL TRP ILE ARG THR PRO PRO ALA TYR ARG PRO PRO ASN ALA PRO ILE LEU SER
TTC GGA GTG TGG ATT CGC ACT CCT CCA GCC TAT AGA CCA CCA AAT GCC CCT ATC TTA TCA

          520                           540                           560
THR LEU PRO GLU THR THR VAL VAL ARG ARG ARG ASP ARG GLY ARG SER PRO ARG ARG ARG
ACA CTT CCG GAA ACT ACT GTT GTT AGA CGA CGG GAC CGA GGC AGG TCC CCT AGA AGA AGA

          580                           600                           620
THR PRO SER PRO ARG ARG ARG ARG SER PRO SER PRO ARG ARG ARG ARG SER GLN SER ARG
ACT CCC TCG CCT CGC AGA CGC AGA TCT CCA TCG CCG CGT CGC AGA AGA TCT CAA TCT CGG

          640                           660                           680
GLU SER GLN CYS END                     END
GAA TCT CAA TGT TAG TAT TCC TTG GAC TCA TAA GGT GGG AAA CTT TAC GGG GCT TTT ATT

          700
                                    END
CTC TAC AGT ACC TAT CTT TAA TCC TGA ATG
```

# FIG. 3

CONSTRUCTION OF RECOMBINANT DNA CLONE 12.88b FOR HBcAg PRODUCTION

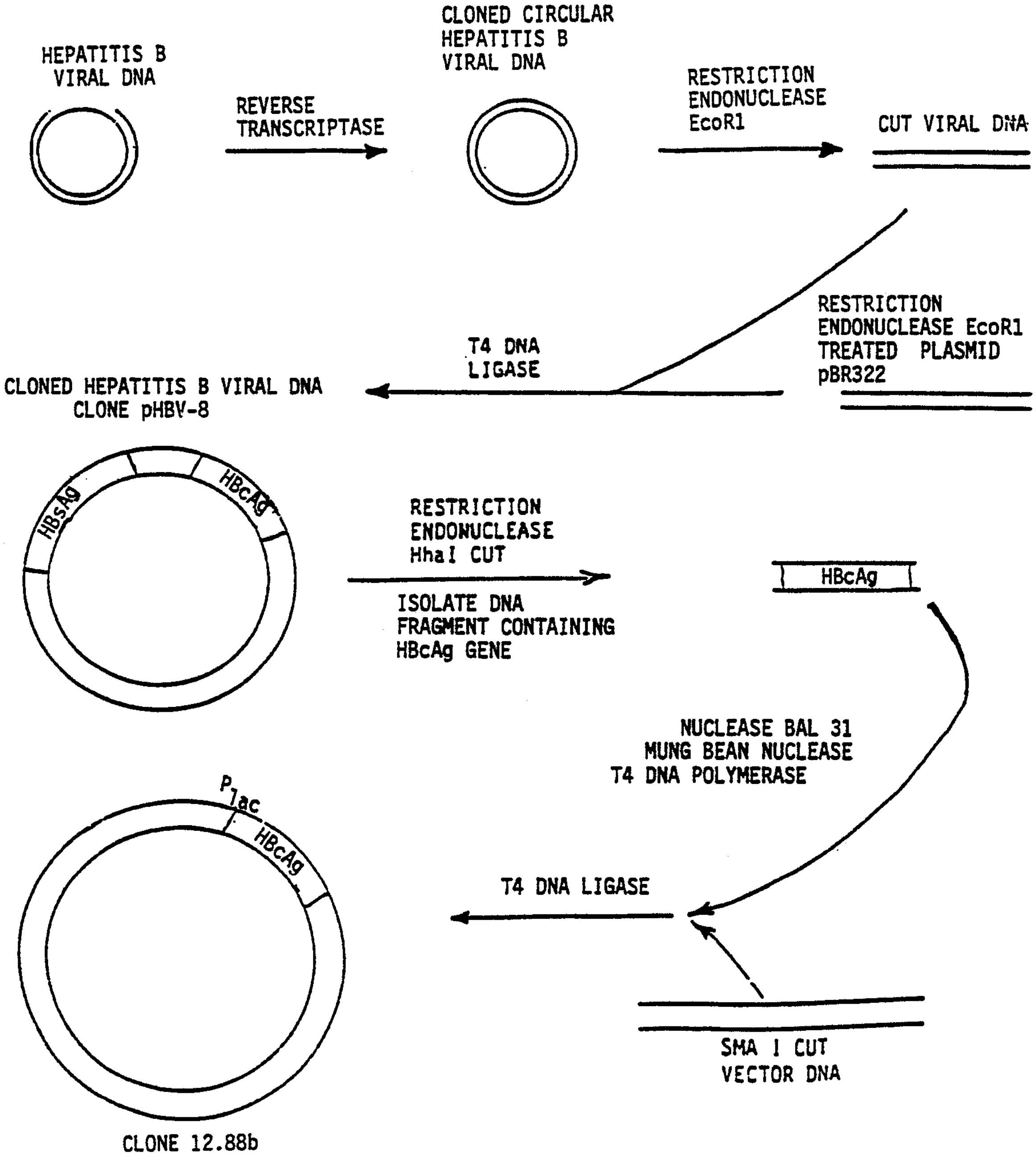

FIG. 4

**CONSTRUCTION OF CLONES EXPRESSING HBcAg IN E.COLI**

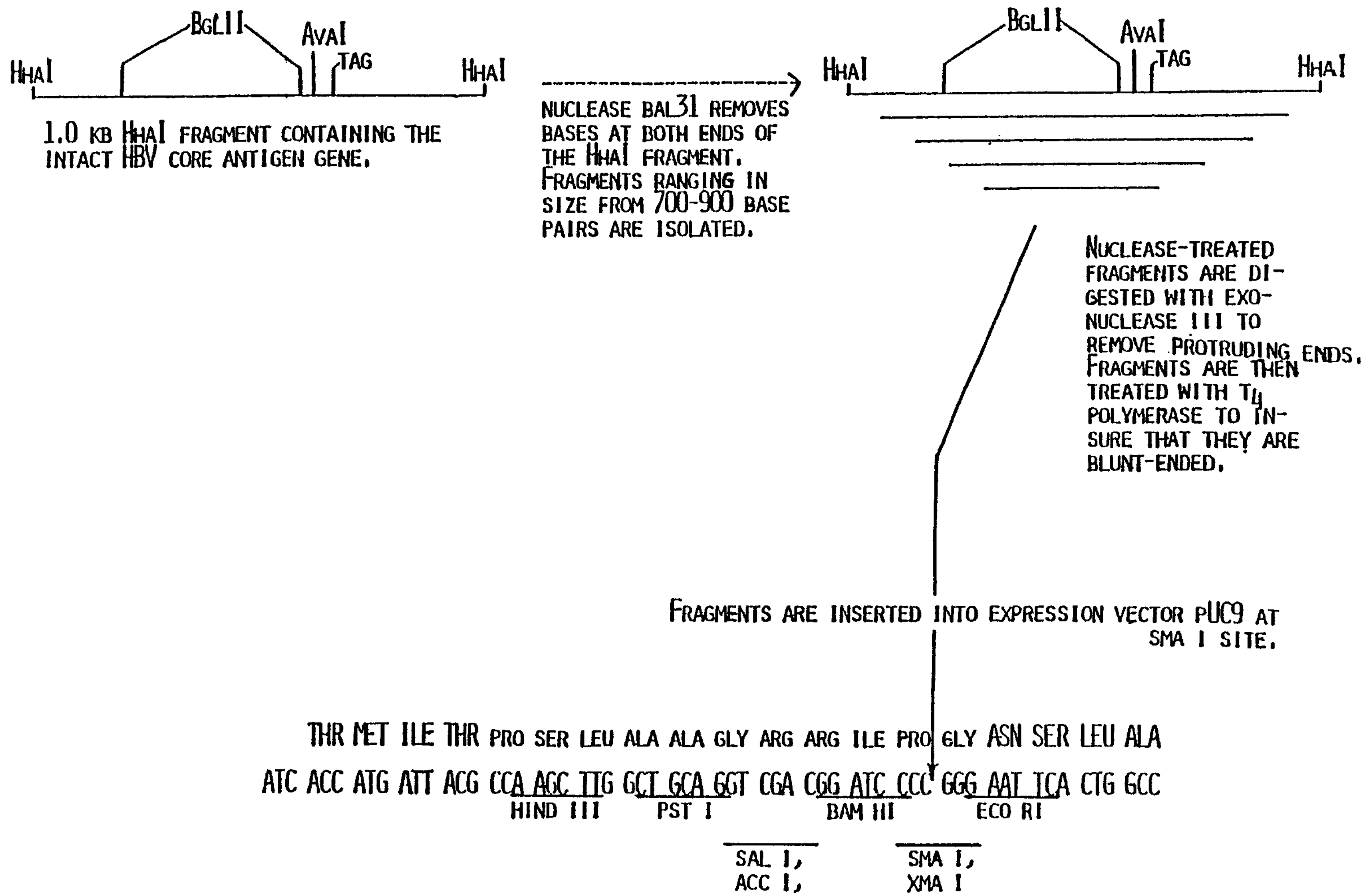

# FIG. 5

DNA And Amino Acid Sequence of HBcAg Produced by Clone 12.88b

```
----BACTERIAL β-GALACTOSIDASE LEADER SEQUENCE----------|HBV DNA Insert start
                                       10              |                    20
Thr Met Ile Thr Pro Ser Leu Ala Ala Gly Arg Arg Ile Pro|Trp Leu Trp Gly Met Asp
ACC ATG ATT ACG CCA AGC TTG GCT GCA GGT CGA CGG ATC CCC TGG CTT TGG GGC ATG GAC

                                       30                                      40
Ile Asp Pro Tyr Lys Glu Phe Gly Ala Thr Val Glu Leu Leu Ser Phe Leu Pro Ser Asp
ATT GAC CCT TAT AAA GAA TTT GGA GCT ACT GTG GAG TTA CTC TCG TTT TTG CCT TCT GAC

                                       50                                      60
Phe Phe Pro Ser Val Arg Asp Leu Leu Asp Thr Ala Ser Ala Leu Tyr Arg Glu Ala Leu
TTC TTT CCT TCC GTC AGA GAT CTC CTA GAC ACC GCC TCA GCT CTG TAT CGA GAA GCC TTA

                                       70                                      80
Glu Ser Pro Glu His Cys Ser Pro His His Thr Ala Leu Arg Gln Ala Ile Leu Cys Trp
GAG TCT CCT GAG CAT TGC TCA CCT CAC CAT ACT GCA CTC AGG CAA GCC ATT CTC TGC TGG

                                       90                                     100
Gly Glu Leu Met Thr Leu Ala Thr Trp Val Gly Asn Asn Leu Glu Asp Pro Ala Ser Arg
GGG GAA TTG ATG ACT CTA GCT ACC TGG GTG GGC AAT AAT TTG GAA GAT CCA GCA TCT AGG

                                      110                                     120
Asp Leu Val Val Asn Tyr Val Asn Thr Asn Met Gly Leu Lys Ile Arg Gln Leu Leu Trp
GAC CTT GTA GTA AAT TAT GTT AAT ACT AAC CTG GGT TTA AAG ATC AGG CAA CTA TTG TGG

                                      130                                     140
Phe His Ile Ser Cys Leu Thr Phe Gly Arg Glu Thr Val Leu Glu Tyr Leu Val Ser Phe
TTT CAT ATA TCT TGC CTT ACT TTT GGA AGA GAG ACT GTA CTT GAA TAT TTG GTC TCT TTC

                                      150                                     160
Gly Val Trp Ile Arg Thr Pro Pro Ala Tyr Arg Pro Pro Asn Ala Pro Ile Leu Ser Thr
GGA GTG TGG ATT CGC ACT CCT CCA GCC TAT AGA CCA CCA AAT GCC CCT ATC TTA TCA ACA

                                      170                                     180
Leu Pro Glu Thr Thr Val Val Arg Arg Arg Asp Arg Gly Arg Ser Pro Arg Arg Arg Thr
CTT CGG GAA ACT ACT GTT GTT AGA CGA CGG GAC CGA GGC AGG TCC CCT AGA AGA AGA ACT

                                      190                                     200
Pro Ser Pro Arg Arg Arg Arg Ser Pro Ser Pro Arg Arg Arg Arg Ser Gln Ser Arg Glu
CCC TCG CCT CGC AGA CGC AGA TCT CCA TCG CCG CGT CGC AGA AGA TCT CAA TCT CGG GAA

Ser Gln Cys END --------------Non coding HBV DNA--------------->
TCT CAA TGT TAG TAT TCC TTG GAC TCA TAA GGT GGG AAA CTT TAC GGG GCT TTA TTC CTC
```

FIG. 6

CONSTRUCTION OF DELETION MUTANTS OF CLONE 12.88b

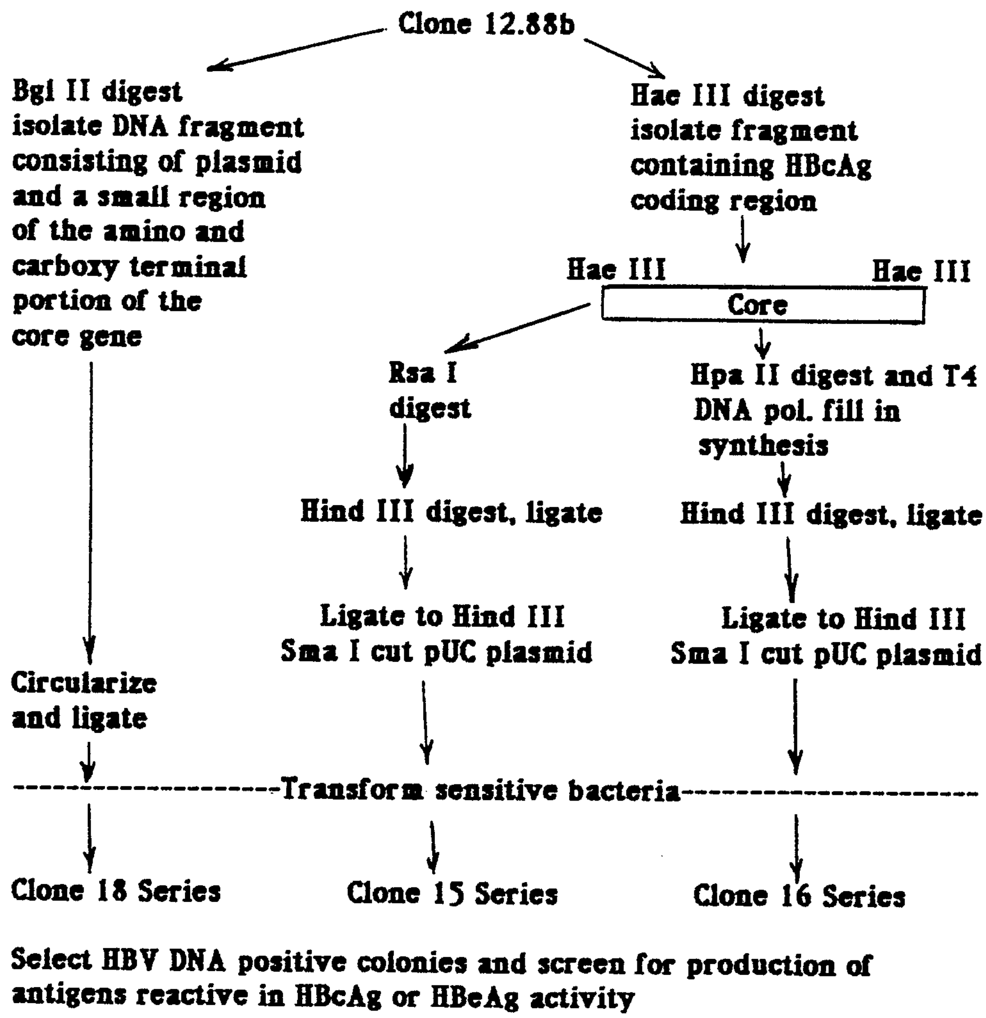

# FIG. 7

COMPARISON OF DELETION MUTANTS RELATIVE TO CLONE 12.88b

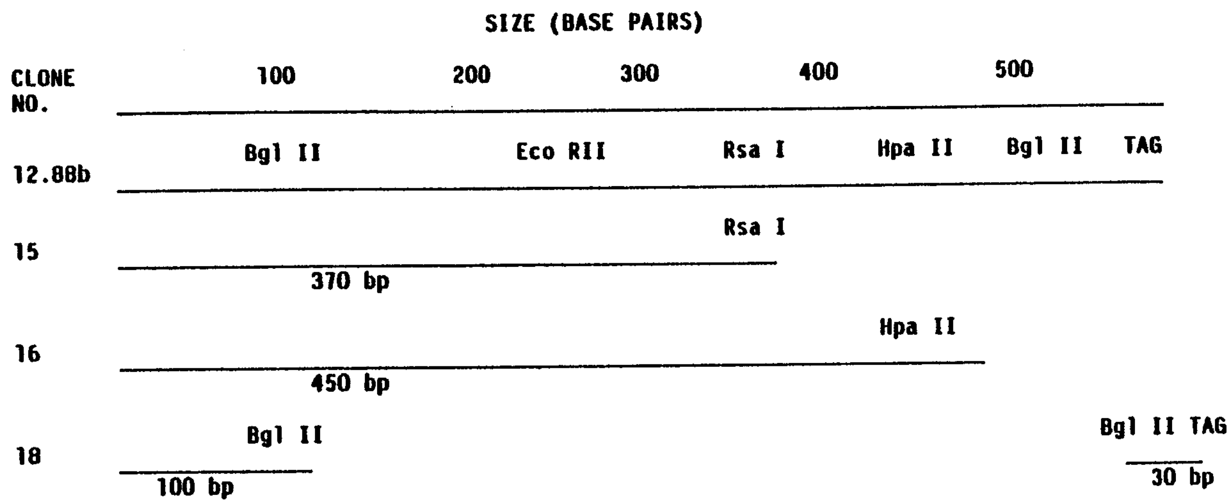

# FIG. 8

DNA And Amino Acid Sequence of HBeAg Produced by Clone 16.4

```
                                                               |
----BACTERIAL β-GALACTOSIDASE LEADER SEQUENCE--------- | HBV DNA Insert Start--
                                     10                  |                    20
Thr Met Ile Thr Pro Ser Leu Ala Ala Gly Arg Arg Ile Pro|Trp Leu Trp Gly Met Asp
ACC ATG ATT ACG CCA AGC TTG GCT GCA GGT CGA CGG ATC CCC|TGG CTT TGG GGC ATG GAC

                                     30                                       40
Ile Asp Pro Tyr Lys Glu Phe Gly Ala Thr Val Glu Leu Leu Ser Phe Leu Pro Ser Asp
ATT GAC CCT TAT AAA GAA TTT GGA GCT ACT GTG GAG TTA CTC TCG TTT TTG CCT TCT GAC

                                     50                                       60
Phe Phe Pro Ser Val Arg Asp Leu Leu Asp Thr Ala Ser Ala Leu Tyr Arg Glu Ala Leu
TTC TTT CCT TCC GTC AGA GAT CTC CTA GAC ACC GCC TCA GCT CTG TAT CGA GAA GCC TTA

                                     70                                       80
Glu Ser Pro Glu His Cys Ser Pro His His Thr Ala Leu Arg Gln Ala Ile Leu Cys Trp
GAG TCT CCT GAG CAT TGC TCA CCT CAC CAT ACT GCA CTC AGG CAA GCC ATT CTC TGC TGG

                                     90                                      100
Gly Glu Leu Met Thr Leu Ala Thr Trp Val Gly Asn Asn Leu Glu Asp Pro Ala Ser Arg
GGG GAA TTG ATG ACT CTA GCT ACC TGG GTG GGC AAT AAT TTG GAA GAT CCA GCA TCT AGG

                                    110                                      120
Asp Leu Val Val Asn Tyr Val Asn Thr Asn Met Gly Leu Lys Ile Arg Gln Leu Leu Trp
GAC CTT GTA GTA AAT TAT GTT AAT ACT AAC ATG GGT TTA AAG ATC AGG CAA CTA TTG TGG

                                    130                                      140
Phe His Ile Ser Cys Leu Thr Phe Gly Arg Glu Thr Val Leu Glu Tyr Leu Val Ser Phe
TTT CAT ATA TCT TGC CTT ACT TTT GGA AGA GAG ACT GTA CTT GAA TAT TTG GTC TCT TTC

                                    150                                      160
Gly Val Trp Ile Arg Thr Pro Pro Ala Tyr Arg Pro Pro Asn Ala Pro Ile Leu Ser Thr
GGA GTG TGG ATT CGC ACT CCT CCA GCC TAT AGA CCA CCA AAT GCC CCT ATC TTA TCA ACA

                                    170                                      180
Leu Pro Glu|Phe Thr Gly Arg Arg Phe Thr Thr Ser
CTT CCG GAA|TTC ACT GGC CGT CGT TTT ACA ACA TCG TGA CTG GGA AAA CCC TGG CGT TAC
           |VECTOR DNA TERMINAL SEQUENCE-------STOP

CCA ACT TAA
    STOP
```

FIG. 9

Anti-HBe RIA
rDNA HBeAg

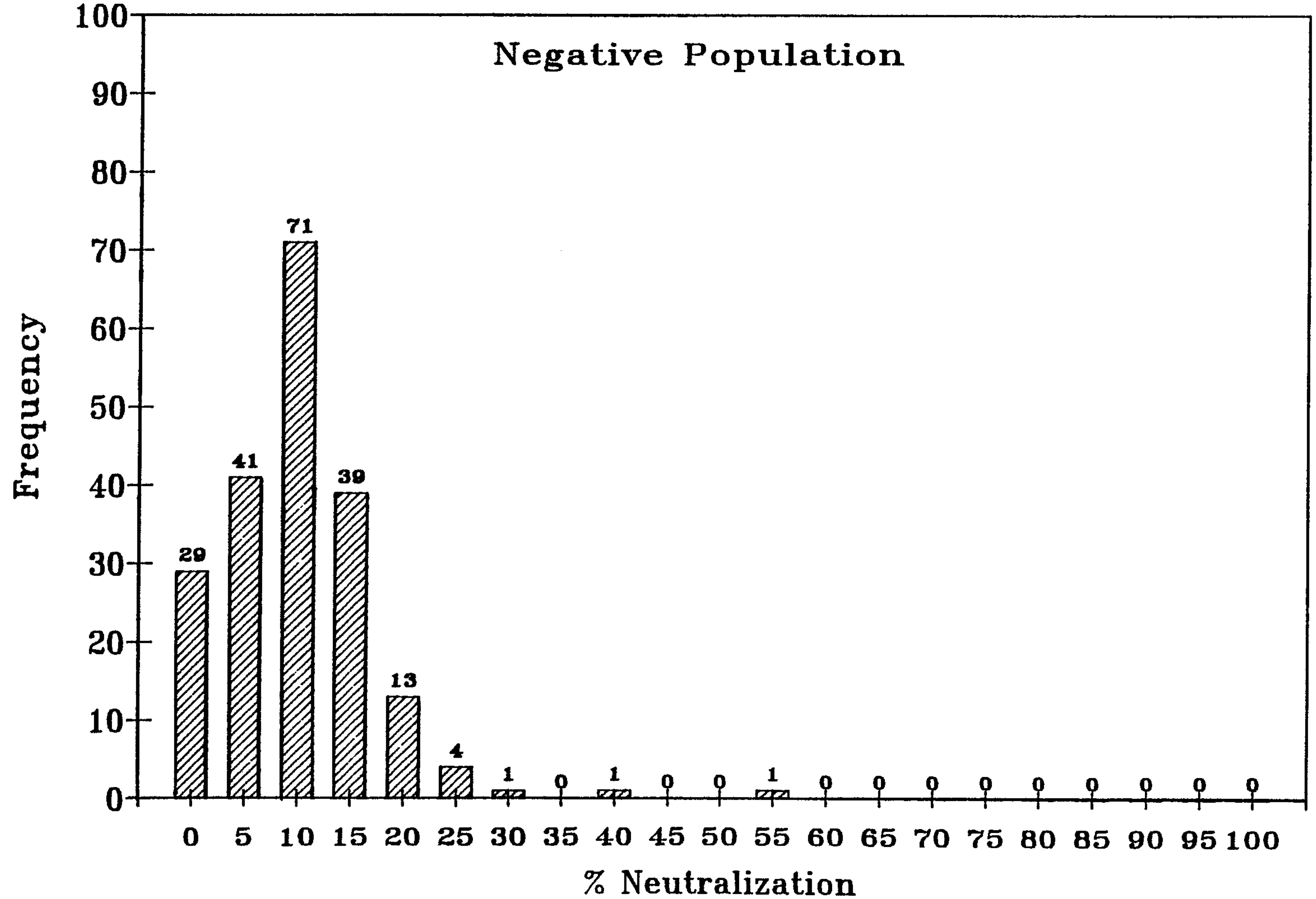

FIG. 10

Anti-HBe RIA

Human Plasma HBeAg

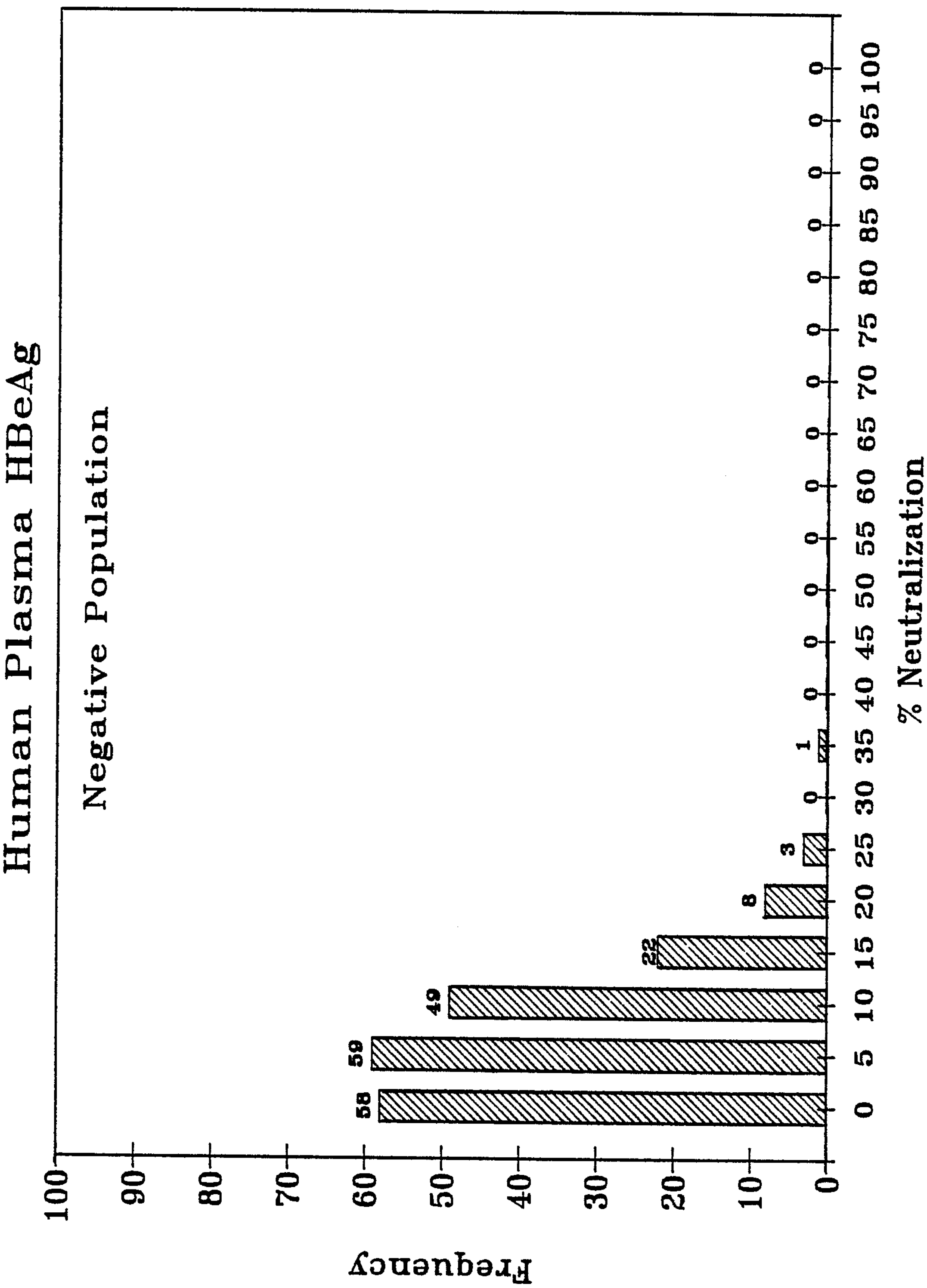

FIG. 11

Anti–HBe EIA
rDNA HBeAg

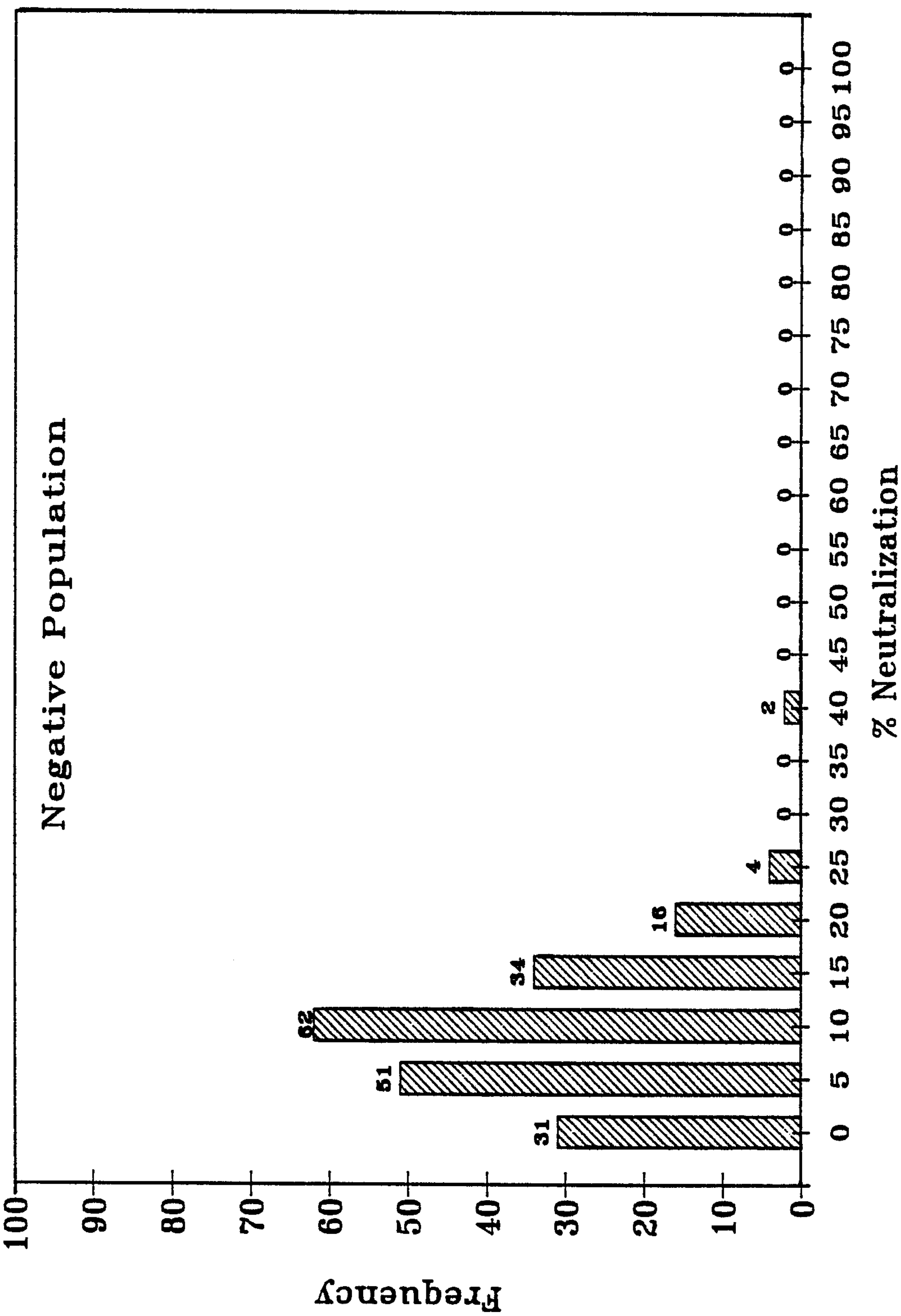

FIG. 12

Anti-HBe EIA
Human Plasma HBeAg

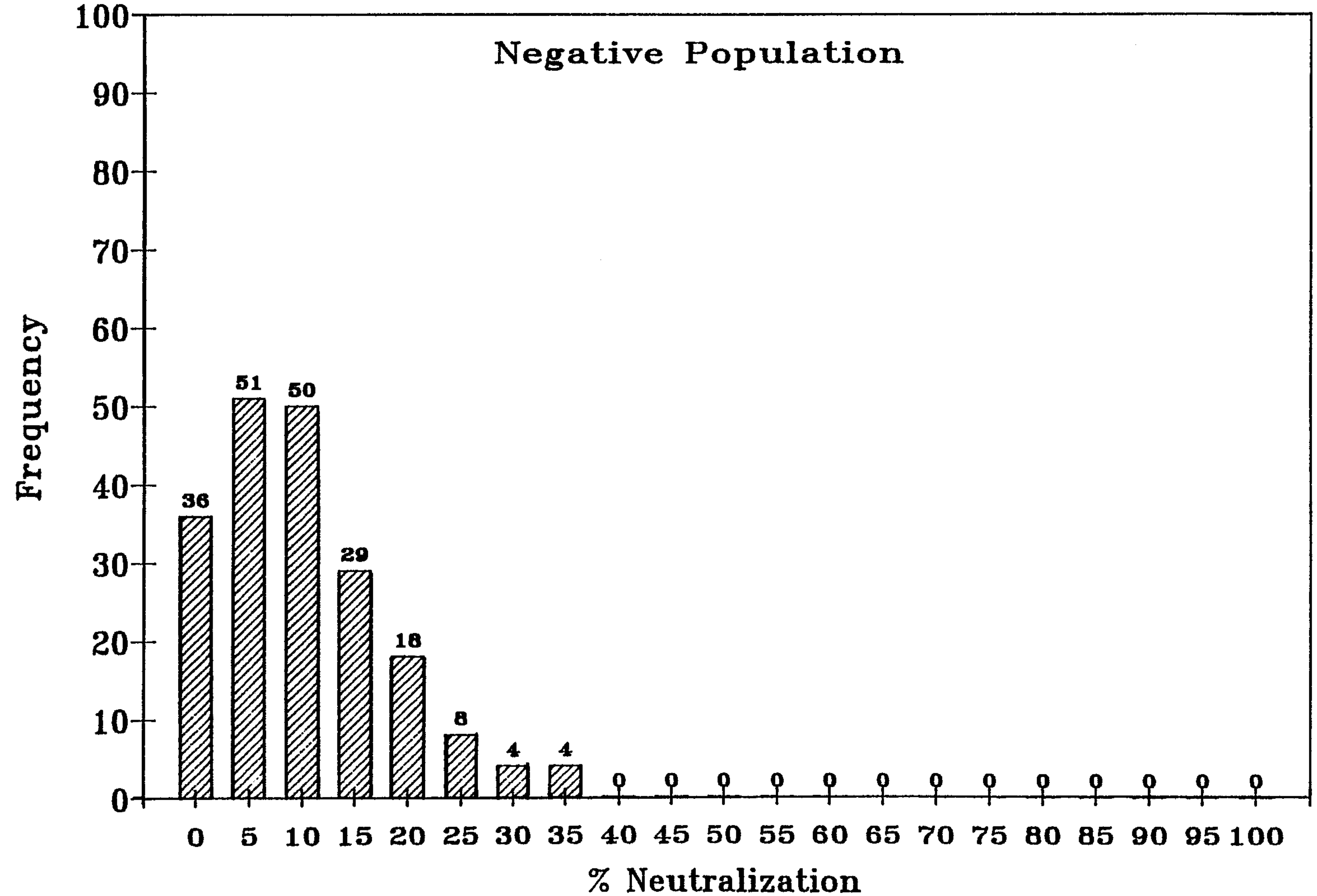

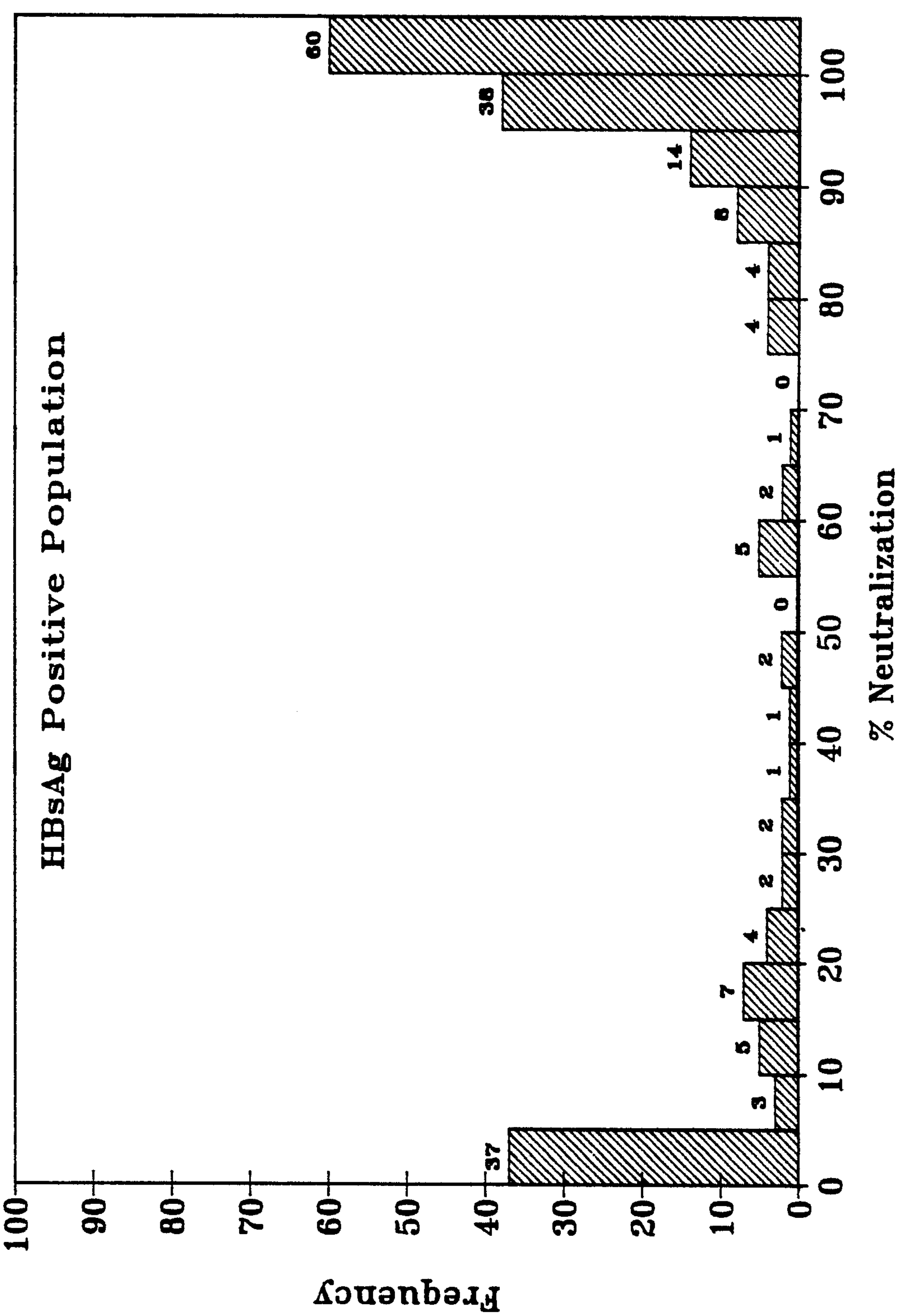


**FIG.13**
Anti–HBe RIA
Human Plasma HBeAg

FIG. 14

Anti-HBe RIA
rDNA HBeAg

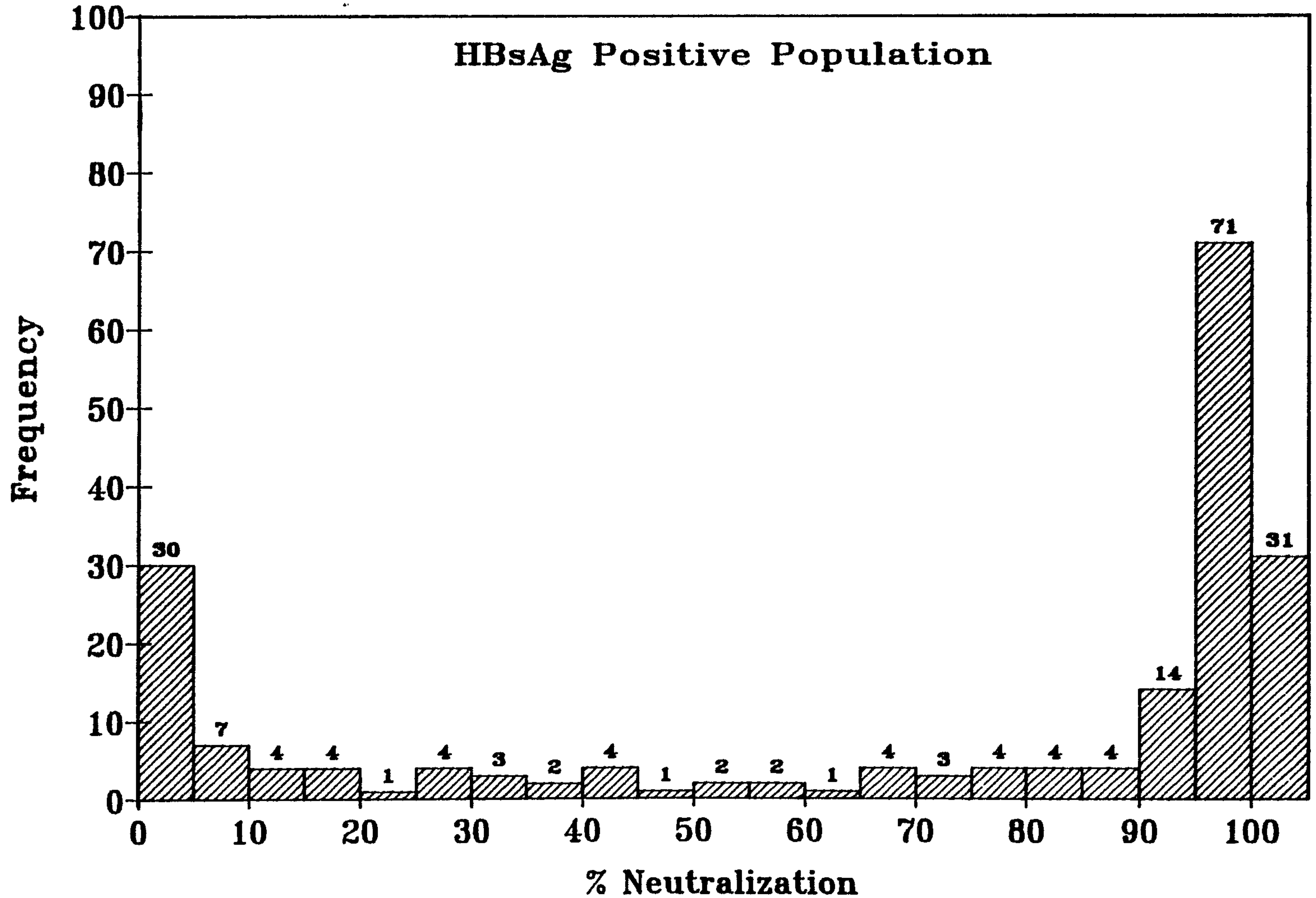

FIG. 15

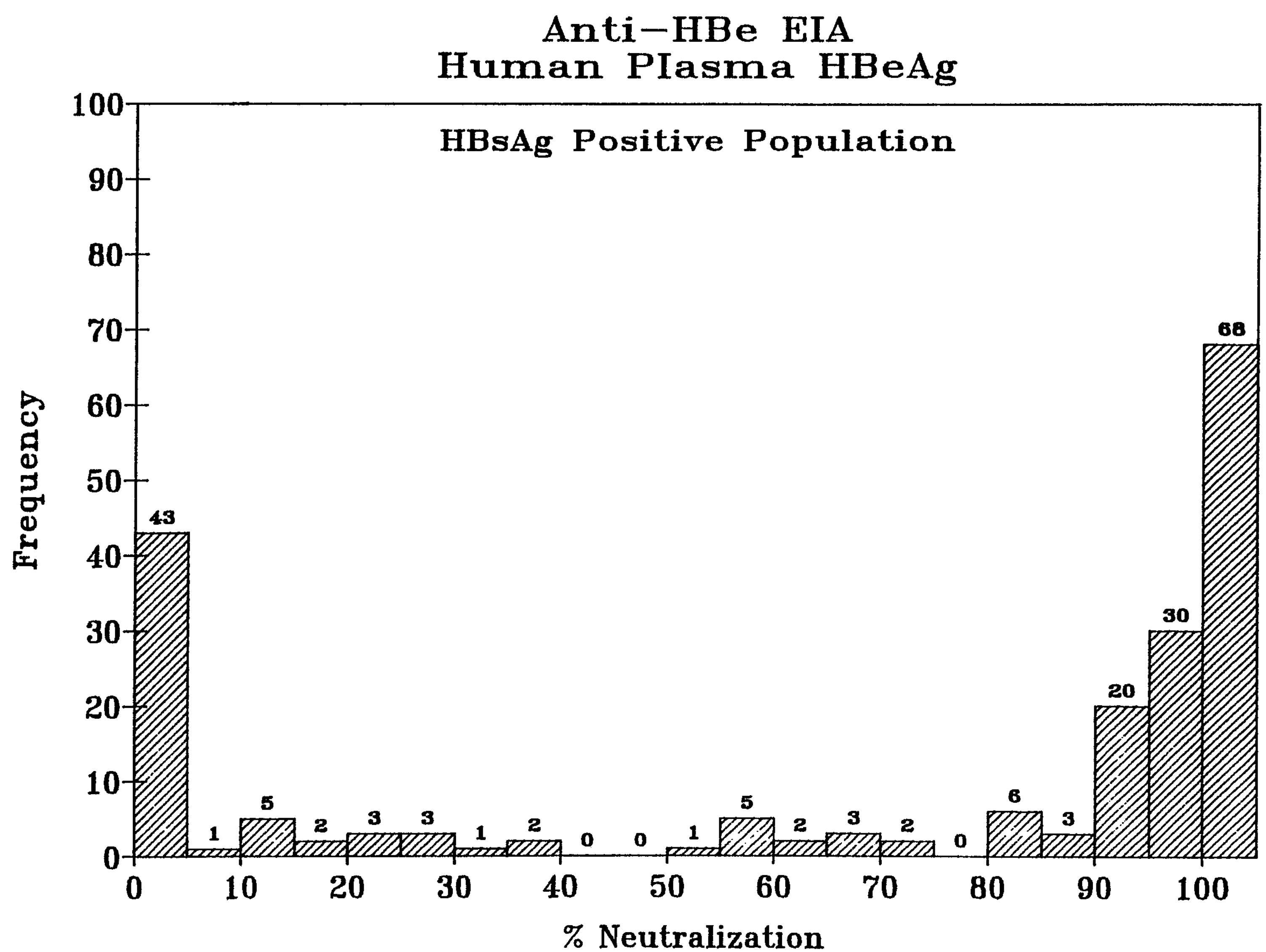
Anti–HBe EIA
Human Plasma HBeAg
HBsAg Positive Population
Frequency
100
90
80
70
60
50
40
30
20
10
0
43
1
5
2
3
3
1
2
0
0
1
5
2
3
2
0
6
3
20
30
68
0
10
20
30
40
50
60
70
80
90
100
% Neutralization

FIG.16

Anti–HBe EIA
rDNA HBeAg

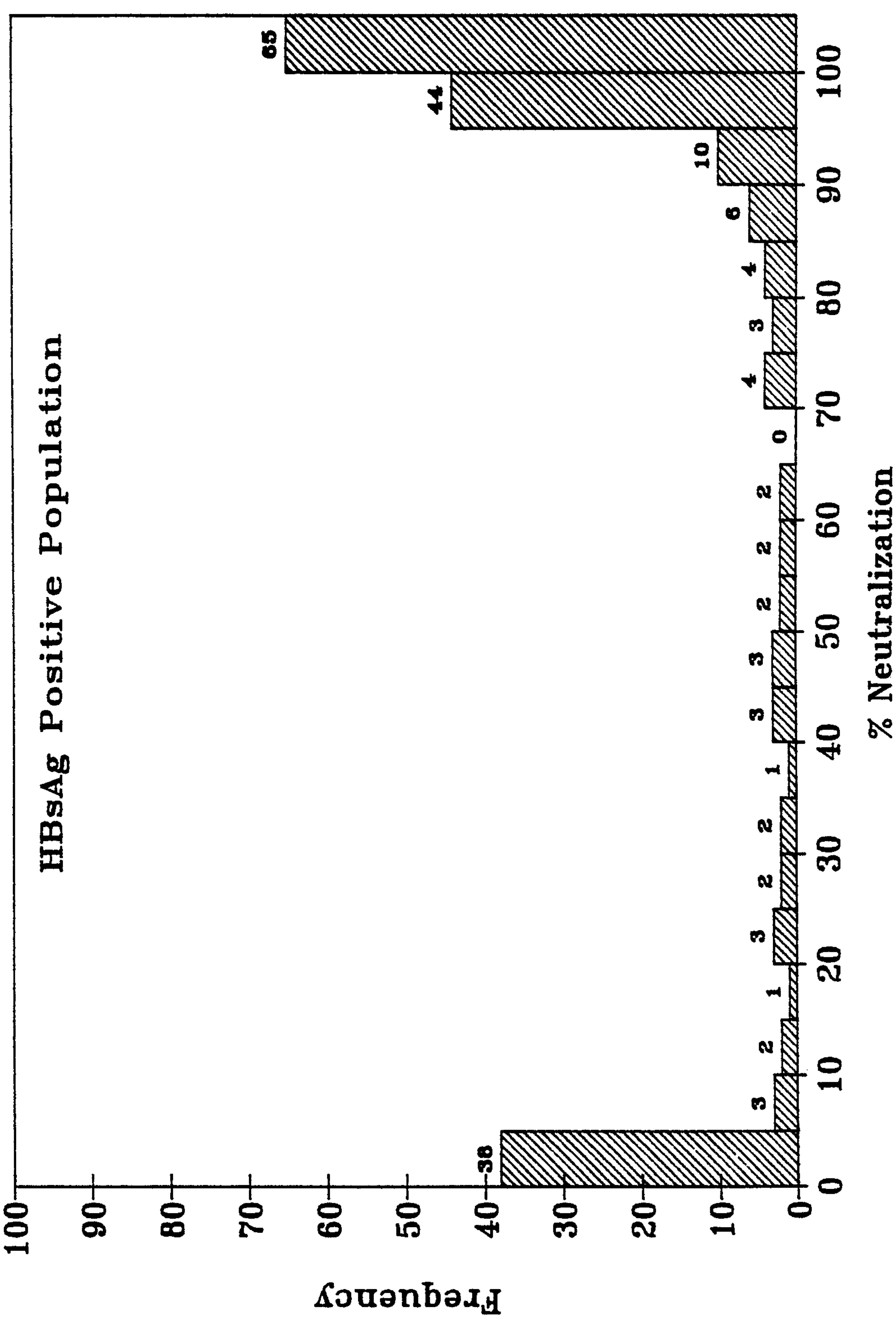

European Patent Office

# EUROPEAN SEARCH REPORT

Application Number

EP 87 11 7370

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X O | ABSTRACTS OF PAPERS PRESENTED AT THE 1986 MEETING ON MOLECULAR BIOLOGY OF HEPATITIS B VIRUSES, 28th-31st August 1986, abstract no. 25, Cold Spring Harbour Laboratory, Cold Spring Harbour, New York, US; X.K. MA et al.: "Effect of pre-core and portion of core sequence on the expression of HBcAg and HBeAg in E.coli" * Abstract * --- | 1-7,14-23 | C 12 N 15/00<br>G 01 N 33/576<br>A 61 K 39/29 |
| D,Y | EP-A-0 075 395 (BIOGEN N.V.) * Page 16, example 3; claims 9-18 * --- | 1-7,14-23 | |
| D,Y | THE JOURNAL OF IMMUNOLOGY, vol. 130, no. 6, June 1983, pages 2903-2906, The American Association of Immunologists, US; K. TAKAHASHI et al.: "Immunochemical structure of hepatitis B e antigen in the serum" * Page 2904, column 1, lines 41-58; page 2905, column 2, lines 23-43; page 2906, lines 1-16 * --- | 1-7,14-23 | TECHNICAL FIELDS SEARCHED (Int. Cl.4) |
| Y | BIOLOGICAL ABSTRACTS, vol. 80, 1985, abstract no. 4874; SLUSARCZYK et al.: "Association of hepatitis B e antigen with the core of the hepatitis D virus", & LIVER 5(1), 48-53, 1985 * Abstract * --- | 8-13 | C 12 N<br>A 61 K |
| D,Y | WO-A-8 505 637 (BIOGEN N.V.) * Page 10; page 11, lines 1-6 * --- -/- | 8-13 | |

The present search report has been drawn up for all claims

| Place of search | Date of completion of the search | Examiner |
|---|---|---|
| THE HAGUE | 14-01-1988 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 87 11 7370

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | EP-A-0 218 474 (TAKEDA CHEMICAL INDUSTRIES) * Whole document; especially, page 3, lines 28-32; examples 6,8 * --- | 1-7,14 | |
| D,A | JOURNAL OF MEDICAL VIROLOGY, vol. 8, 1981, pages 237-243, Alan R. Liss, Inc.; P. MacKAY et al.: "The conversion of hepatitis B core antigen synthesized in E coli into e antigen" --- | | |
| A | J. GEN. VIROL., vol. 42, 1979, pages 513-519, SGM, GB; H. YOSHIZAWA et al.: "Demonstration of hepatitis B e antigen in hepatitis B core particles obtained from the nucleus of hepatocytes infected with hepatitis B virus" ----- | | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 14-01-1988 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document